# EUROPEAN PATENT SPECIFICATION

(11) **EP 0 858 322 B1**
(45) Date of publication and mention of the grant of the patent: **05.02.2003**
(21) Application number: 96937820.7
(22) Date of filing: 01.11.1996
(51) Int. Cl.: A61K 9/00

(54) **TREATMENT OF A CORONARY CONDITION BY DELIVERY OF THERAPEUTICS TO THE PERICARDIAL SPACE**
BEHANDLUNG VON KORONAREN STÖRUNGEN DURCH ARZNEIMITTELABGABE IM HERZBEUTEL
TRAITEMENT DES AFFECTIONS CORONARIENNES PAR INTRODUCTION D'UN AGENT THERAPEUTIQUE DANS L'ESPACE PERICARDIQUE

(30) Priority: 01.11.1995 US 7158 P; 28.10.1996 US 738848
(43) Date of publication of application: 19.08.1998
(60) Divisional application: 02014430.9
(73) Proprietor: CHIRON CORPORATION, Emeryville, California 94608 (US)
(72) Inventor: HUNG, David, T., San Francisco, CA 94127 (US)
(74) Representative: Goldin, Douglas Michael
(86) International application number: US9617411
(87) International publication number: WO97016170

(56) References cited:
- WO-A-92/10221
- WO-A-96/00112
- WO-A-96/39830
- US-A- 5 269 326
- AMERICAN HEART JOURNAL, vol. 129, no. 5, May 1995, pages 924-931, XP000618320 C. LANDAU ET AL: "Intrapericardial basic fibroblast growth factor induces myocardial angiogenesis in a rabbit model of chronic ischemia"
- GENE THERAPY, vol. 1, 1994, pages 51-58, XP000563621 E. BARR ET AL: "Efficient catheter-mediated gene transfer into the heart using replication-defective adenovirus" cited in the application
- CIRCULATION, vol. 91, no. 2, 15 January 1995, pages 541-548, XP000568740 E. G. NABEL : "Gene Therapy for Cardiovascular Disease"

## Description

### Field of the Invention

The invention relates to the delivery of therapeutic agents to the pericardial space for the treatment of a variety of coronary conditions and cardiovascular indications. The therapeutic agents used in the invention include polypeptides, polynucleotides, and other drugs. The pharmaceutical compositions for delivery of the therapeutic agents include a variety of buffers, excipients, liposomes, particles, polymers, gels, matrices for facilitating effective delivery to the pericardial space. The pericardial delivery can be by an internal entry, such as through an atrium or ventricle, or can be by an external entry through the chest wall.

### Background of the Invention

Presently, most cardiovascular therapeutics are delivered either orally or intravascularly, by percutaneous cannulation of the systemic venous systems, i.e. standard intravenous therapy. With specialized catheters and delivery devices, cardiovascular therapeutics can be also delivered close to or directly into the heart, through cannulation of the great venous structures (i.e. superior or inferior vena cava, subclavian vein, etc.) or cannulation of the coronary arterial or pulmonary arterial circulation. Because oral or intravascular drug delivery generally results in significant systemic exposure to the agent being delivered, many past methods of drug delivery to the heart have either been limited by complications of systemic toxicity, or have employed suboptimal amounts of therapeutic agents in order to avoid systemic toxicity. A few of the classes of drugs that have been delivered by the above methods to treat a variety of cardiovascular conditions and have been known to result in systemic toxicity include anti-arrythmics, calcium channel blockers, beta-blockers, contractility improving agents, afterload reducers, preload reducers, vasoactive agents, immunosuppressive agents, antibiotics, and anti-inflammatory agents. Specific examples of drugs in each of the above categories, respectively, include amiodarone, verapamil, propranolol, digitalis, hydralazine, nitroglycerin, cyclosporin, isoniazid, and prednisone.

In the treatment of coronary artery restenosis, following percutaneous transluminal coronary angioplasty, the local delivery of anti-restenosis agents directly into the coronary vasculature, either at the endoluminal surface or within the coronary vessel wall via a variety of catheters and coated stents, has become increasingly popular. The basic premise for such delivery is that it allows higher concentrations and more prolonged administration of these agents to be achieved at the pathological site than is practical or achievable with systemic administration. Local drug delivery thereby offers the potential benefits of increasing therapeutic efficacy while reducing systemic toxicity or, in other words, increasing the "therapeutic index" of the delivered agent. However, because the delivery of therapeutic agents to the endoluminal surface of the coronary vasculature generally subjects these agents to the vigorous blood flow of the coronary artery, the retention of these agents at the desired site is often problematic. Drug-impregnated gels or polymers deposited at the endoluminal surface of coronary vessels can prolong drug retention at the desired site but generally suffer from a relatively limited drug storage capacity and, in addition, pose at least theoretical problems regaled to the gels or polymers themselves such as fragmentation, distal embolization, and inflammation, among other concerns Prolonged retention of therapeutic agents deposited within the coronary vessel wall itself is hampered by many obstacles including dissipation by drainage by either passive diffusion or via vaso vasorum from the coronary vessel wall and also by the limited capacity of the vessel wall as a drug depot site.
Intrapericardial administration of basic fibroblast growth factor has been shown to induce myocardial angiogenesis in a rabbit model of chronic ischemia (Landau *et al*., American Heart Journal, vol 129, pp 924-931, 1995). Pericardial administration of therapeutic agents has also been disclosed in WO 96/00112 and WO 96/39830 both of which were published after the first claimed priority date of the present application.
It would be advantageous to develop other methods of treatment of patients having cardiovascular conditions that reduce adverse side effects and heighten efficacy.

### Summary of the Invention

The present invention provides use of a therapeutic agent in the manufacture of a pharmaceutical composition for treatment or prevention of a coronary condition wherein the pharmaceutical composition is administered to the pericardial space of a patient simultaneously with, or sequentially after enhancing access of the first therapeutic agent to myocardial tissue.

The invention also provides use of a first therapeutic agent in the manufacture of a pharmaceutical composition for treatment or prevention of a coronary condition wherein the pharmaceutical composition is administered to the pericardial space of a patient and the pharmaceutical composition comprises an agent which enhances access to myocardial tissue.

The invention further provides use of a therapeutic agent in the manufacture of a pharmaceutical composition for use in a method of treating cardiac muscle tissue which method comprises:
a. identifying an infarct or ischemic zone,
b. accessing a pericardial space in the region of the infarct or ischemic zone; and
c. delivering the pharmaceutical composition to the region of the infarct or ischemic zone,
wherein access of the therapeutic agent to the region of the infarct or ischemic zone is enhanced by creating perfusion channels, administering a proangiogenic factor to the pericardial space or including in the pharmaceutical composition an agent that increases tissue penetration of the therapeutic agent.

The invention yet further provides use of an agent capable of lysing a pericardial/epicardial adhesion for the manufacture of a medicament for use in a method of accessing the pericardial space prior to administration of a therapeutic agent to the pericardial space, wherein the method of accessing the pericardial space further comprises the step of expanding the pericardial space.

The therapeutic agents used in the invention may be polypeptides, polynucleotides or other drugs. Such agents may be encapsulated in liposomes, including any liposome composition such as cyclodextrin liposomes, heterovesicular liposomes, DepoFoam® and synthetic membrane vesicles. It is also contemplated by the invention that the drug or polynucleotide is delivered in other formulations commonly known in the art, including buffers, excipients, polymers, gels, including for example biodegradable gels or Focalgel®, and other matrices.

It is a further object of the invention that the therapeutic agent that is administered into the pericardial space, whether a polypeptide, polynucleotide, or other drug or combination of one or more of these agents, be one of the following: an anti-apoptotic agent, a thrombolytic agent, a pro-angiogenic agent, a complement blocker, an inhibitor of reperfusion injury, an anti-arrhythmic agent, a contractility improving agent, a myocyte growth factor, a vasoactive agent, an anti-hypertensive agent, a calcium channel blocker, a beta-blocker, an afterload reducer, a preload reducer, a vasoactive agent, an anti-inflammatory agent, an immunomodulating or immunosuppressive agent, a free radical scavenging agent, an inhibitor of reactive oxygen metabolites, an anti-thrombotic agent, an anti-platelet agent, an anti-integrin agent, an anti-proliferative agent, a pro-apoptotic agent, an anti-angiogenic agent, and antibiotics, including anti-bacterial, anti-viral, anti-fungal, and anti-parasitic agents, and antitumor agents, including chemotherapeutic agents, radiation sensitizers, and radioactive implants, and a biologically active fragment or chimera of one of these molecules.

Employment of the invention has the further object to prevent, treat, or reduce the attendant effects or complications of the following coronary or cardiovascular conditions:
1) atherosclerosis, and conditions that predispose to pathological atherosclerotic plaque development in the coronary arteries including:
   - lipid/cholesterol deposition,
   - macrophage/inflammatory cell recruitment,
   - plaque rupture,
   - thrombosis,
   - platelet deposition, and
   - neointimal proliferation;
2) ischemic syndromes and attendant syndromes, including but not limited to:
   - myocardial infarction,
   - stable and unstable angina,
   - coronary artery restenosis following percutaneous transluminal coronary angioplasty, and
   - reperfusion injury;
3) cardiomyopathies, including but not limited to cardiomyopathies caused by or associated with:
   - ischemic syndromes,
   - cardiotoxins such as alcohol and chemotherapeutic agents like adriamycin,
   - infections, such as viral (CMV) and parasitic, such as caused by Trypanosoma cruzi,
   - hypertension,
   - metabolic diseases, including but not limited to uremia, beriberi, glycogen storage disease,
   - radiation,
   - neuromuscular disease, such as Duchenne's muscular dystrophy,
   - infiltrative diseases (including but not limited to sarcoidosis, hemochromatosis, amyloidosis, Fabry's disease, Hurler's syndrome,
   - trauma, and
   - idiopathic causes;
4) a/dysrrhythmias, including but not limited to a/dysrrhythmias resulting from the same causes listed above for cardiomyopathies;
5) infections, including bacterial, viral, fungal, and parasitic causes;
6) cardiac tumors;
7) inflammatory conditions, including but not limited to myocarditis, pericarditis, endocarditis, immune cardiac rejection and conditions resulting from idiopathic, autoimmune, or connective tissue diseases; and
8) hypertension.

It is a further object of the invention that where the agent used for treatment of coronary artery occlusion , the agent is selected from an inhibitor of lipid or cholesterol synthesis or deposition, such as fish oil, HMG, an inhibitor of macrophage or inflammatory cell recruitment or activation, such as NF-κB inhibitors like IκB, pyrolidine dithiocarbamate (PDTC), and N-acetyl cysteine (NAC), microtubule inhibitors like colchicine and Taxol, anti-inflammatory agents such as those mentioned below, an anti-thrombotic agent as described below under treatment of ischemic syndromes, an antiplatelet agent as described below under treatment of ischemic syndromes, and an inhibitor of neointimal proliferation as described below under anti-proliferative agents.

It is another object of the invention that, where the agent used is directed to the prevention, treatment, or reduction of attendant effects of the myocardial ischemic syndromes, the agent is selected from an anti-apoptotic agent, such as tissue plasminogen activator (TPA), an inhibitor of interleukin 1b converting enzyme; a thrombolytic agent, such as urokinase plasminogen activator (UPA), urokinase, streptokinase, inhibitors of α2 plasmin inhibitor, and inhibitors of plasminogen activator inhibitor-1; a pro-angiogenic agent, such as basic and acidic fibroblast growth factor, FGF-5, vascular endothelial growth factor, angiogenin, transforming growth factor alpha and beta, tumor necrosis factor alpha, platelet derived growth factor, placental growth factor, hepatocyte growth factor, and proliferin; a complement blocker, such as decay accelerating factor; an inhibitor of reperfusion injury, such as CAB-2; a calcium channel blocker, such as diltiazem; a beta-blocker, such as propranolol; an afterload reducer, such as hydralazine; a preload reducer, such as nitroglycerin; a vasoactive agent such as nitric oxide (NO), a nitric oxide inhibitor, or an inhibitor of NO synthase; an anti-thrombotic agent, such as tissue factor pathway inhibitor (TFPI), heparin, hirudin, protein C, protein S, anti-thrombin III, tick anti-coagulant peptide (TAP), and antistasin; an antiplatelet agent, such as a glycoprotein IIb/IIIa antagonist, cyclooxygenase inhibitors like aspirin or non-steroidal anti-inflammatory agents, prostacylin, or agents that increase platelet cAMP; anti-proliferative agents, such as a ribozymes, antisense oligonucleotides, antibodies, protein, peptide, or small molecule inhibitors against c-myb, ras/raf, PI3 kinase, cyclins, or such as suicide proteins/genes like Herpes thymidine kinase or proapoptotic proteins/genes like fas, faf, interleukin 1b converting enzyme; inhibitor of reactive oxygen metabolites, such as superoxide dismutase, N-acetyl cysteine, pyrolidine dithiocarbamate (PDTC), vitamin E derivatives, and metal ion chelators; or an anti-angiogenic agent, such as platelet factor 4, thrombospondin, a tissue inhibitor of a metalloproteinase, prolactin, bFGF soluble receptor, angiostatin, TFG-β, interferon-α, and proliferin-related protein. In all cases above where the agent is a protein, a biologically active fragment or a chimera thereof is also contemplated, as is the expression of the gene encoding the protein.

It is a further object of the invention that where the agent used for treatment is intended to improve or prevent cardiomyopathy, the agent is selected from the same list as for treatment of ischemic syndromes above, since ischemic syndromes are a leading cause of cardiomyopathy. In addition, other agents which may be used to treat or prevent cardiomyopathy from other causes include a contractility improving agent, such as digitalis; a myocyte growth factor, such as insulin-like growth factor 1 (IGF-1), cardioprotective agents, such as Cardioxane; an iron-chelating agent, such as desferoxamine; anti-viral or anti-parasitic agents, a free radical scavenger, such as superoxide dismutase; or the replacement of genes or proteins that may be deficient or downregulated during the development of cardiomyopathy, such as troponin C or the beta-adrenergic receptor. In all cases above where the agent is a protein, a biologically active fragment or a chimera thereof is also contemplated, as is the expression of the gene encoding the protein.

Another object of the invention is that where the agent used for treatment is an anti-arrhythmic agent, the agent is selected from any of numerous known anti-arrhythmics including adenosine, quinidine, propranolol, digoxin, lidocaine, bretylium, amiodarone, and verapamil.

Still another object of the invention is that, where the agent used for treatment is an antibiotic agent, the agent is selected from antibacterial, antivirals anti-fungal, and antiparasitic agents.

Another object of the invention is that, where the agent used for treatment is an antitumor agent, the agent is selected from chemotherapeutic agents or radiation sensitizers or radioactive implants.

A further object of the invention is that, where the agent used for treatment is an anti-inflammatory agent, the agent is selected from anti-inflammatory or immunomodulating agents including, but not limited to, steroids, non-steroidal anti-inflammatory agents, cyclosporin, chemotherapeutic agents, and complement inhibitors.

Still another object of the invention is that, where the agent used for treatment is an anti-hypertensive agent, the agent is selected from any of numerous known anti-hypertensive agents including but not limited to hydralazine, propranolol, atrial naturetic peptide, and endothelin antagonists.

Accordingly, it is an object of the present invention to provide polypeptides, polynucleotides or other drugs for delivery to the pericardial space, in an appropriate formulation, selected from the group of an anti-apoptotic agent, a thrombolytic agent, a pro-angiogenic agent, an anti-arrythmic agent, a contractility improving agent, a complement blocker, an inhibitor of reperfusion injury, an calcium channel blocker, a beta-blocker, an afterload reducer, a preload reducer, a vasoactive agent, an antithrombotic agent, an anti-platelet agent, anti-proliferative agent, an anti-inflammatory agent, an immunomodulating agent, an immunosuppressive agent, an inhibitor of reactive oxygen metabolites, an anti-angiogenic agent, a myocyte growth factor, a vasoactive agent, a cardioprotective agent, an iron-chelating agent, an anti-hypertensive agent, an anti-integrin agent, a pro-apoptotic agent, an anti-viral agent, an anti-parasitic agent, a free radical scavenger, and genes or proteins that may be deficient or down regulated during the development of cardiomyopathy, including troponin C or the beta-adrenergic receptor, and a biologically active fragment or a chimera thereof.

Another object of the invention is to deliver the therapeutic agent into the pericardial space by injection, catheterisation, laser-created perfusion channels, cannulation, particle gun, or pump.

An additional object of the invention is that the access of the therapeutic agents to the myocardial tissue is increased by such steps including, for example, increasing agent penetration of the myocardium and by creating myocardial perfusion channels by laser. It is an object of the invention that increasing penetration of the myocardium is accomplished by first administering to the pericardial space proangiogenic factors to increase vascularisation of myocardial tissue and subsequently administering a therapeutic agent into the pericardial space or specifically formulating agents to increase their tissue penetration.

In accordance with another object of the invention, the efficacy of myocardial revascularisation by laser is augmented by a therapeutic agent, such as a proangiogenic agent like bFGF, by administration into the pericardial space in close proximity to the laser revascularisation procedure.

Another embodiment includes that the first therapeutic agent is a polypeptide, polynucleotide, a small organic molecule, a peptide, or a peptoid.

Another embodiment is that the first therapeutic agent is a fibroblast growth factor polypeptide (FGF), or an insulin-like growth factor-I polypeptide (IGF-I).

Another embodiment is that the pharmaceutical composition for treating the coronary condition includes also a therapeutically effective amount of a second therapeutic agent, and that the second therapeutic agent is a polypeptide, a polynucleotide, a small organic molecule, a peptide, or a peptoid. The second therapeutic agent thus, can be a fibroblast growth factor polypeptide (FGF), or insulin-like growth factor-I polypeptide (IGF-I).

Administration to the pericardial space may be by internal entry or external entry. Internal entry includes entry through the left atrium, entry through the right ventricle, or entry through the left ventricle. External entry includes open chest procedure, minimally invasive surgery (MIS), or percutaneous entry facilitated by a device including a needle, catheter, cannula, or trocar.

Administration of the pericardial space may be by injection, catheterisation, laser-created perfusion channels, cannulisation, a particle gun, or a pump.

Additionally, yet another embodiment is that any of the pharmaceutical compositions used in the invention can be a liposome, cyclodextrin liposome, heterovesicular liposome, a synthetic membrane vesicle, a gel, a polymer, an excipient, matrices, a charged particle or a buffer.

Further additionally, any of therapeutic agents used in the invention can be an anti-apoptotic agent, a thrombolytic agent, a pro-angiogenic agent, an anti-arrythmic agent, a contractility improving agent, a complement blocker, an inhibitor of reperfusion injury, a calcium channel blocker, a beta-blocker, an afterload reducer, a preload reducer, a vasoactive agent, an anti-thrombotic agent, an anti-platelet agent, anti-proliferative agent, an anti-inflammatory agent, an immunomodulating agent, an immunosuppressive agent, an inhibitor of reactive oxygen metabolites, an anti-angiogenic agent, a myocyte growth factor, a vasoactive agent, a cardioprotective agent, an iron-chelating agent, an anti-hypertensive agent, an anti-integrin agent, a pro-apoptotic agent, an anti-viral agent, an anti-parasitic agent, a free radical scavenger, an anti-tumour agent, and a protein that may be deficient or down regulated during development of cardiomyopathy, or a biologically active derivative thereof.

Further, yet another embodiment of the invention is that any therapeutic agent of the invention can be a polypeptide tissue plasminogen activator (tPA), an inhibitor of interleukin 1β converting enzyme, urokinase plasminogen activator (uPA), urokinase, streptokinase, an inhibitor of α2 plasmin inhibitor, an inhibitor of plasminogen activator inhibitor-1 (PAI-1), basic fibroblast growth factor (bFGF), acidic fibroblast growth factor (aFGF), vascular endothelial cell growth factor (VEGF), angiogenin, transforming growth factor α (TGF-α), transforming growth factor β(TGF-β), tumor necrosis factor-α (TNF-α), platelet derived growth factor (PDGF), placental growth factor (PGF), hepatocyte growth factor, proliferin, decay accelerating factor, CAB-2, tissue factor pathway inhibitor (TFPI), heparin, hirudin, protein C, protein S, anti-thrombin III, tick anti-coagulant peptide (TAP), anti-stasin, glycoprotein IIb/IIa antagonist, antibodies, Herpes thymidine kinase, fas, faf, platelet factor 4, thrombospondin, a tissue inhibitor of a metalloproteinase, prolactin, bFGF soluble receptor, a proliferin-related protein, myocyte growth factor, superoxide dismutase (SOD), troponin C, beta-adrenergic receptor, insulin-like growth factor I (IGF-I), nematode anti-coagulant protein (NAP), biologically active fragments thereof, or chimeras thereof.

Another embodiment of the invention provides more complete access to the heart through administration of a therapeutic agent to a pericardial space by administering an agent capable of lysing a pericardial/epicardial adhesion, and expanding the pericardial space. Another embodiment of the invention is that the agent capable of lysing a pericardial/epicardial adhesion is any fibrinolytic agent, tissue plasminogen activator (tPA), streptokinase, urokinase, collagenase, or a matrix metalloprotease., and that expanding the pericardial space is temporary and accomplished by administration of liquid or air.

Another embodiment is use of a polypeptide therapeutic agent including an anti-apoptotic agent, a thrombolytic agent, a pro-angiogenic agent, an anti-arrythmic agent, a contractility improving agent, a complement blocker, an inhibitor of reperfusion injury, a calcium channel blocker, a beta-blocker, an afterload reducer, a preload reducer, a vasoactive agent, an anti-thrombotic agent, an anti-platelet agent, anti-proliferative agent, an anti-inflammatory agent, an immunomodulating agent, an immunosuppressive agent, an inhibitor of reactive oxygen metabolites, an anti-angiogenic agent, a myocyte growth factor, a vasoactive agent, a cardioprotective agent, an iron-chelating agent, an anti-hypertensive agent, an anti-integrin agent, a pro-apoptotic agent, an anti-viral agent, an anti-parasitic agent, a free radical scavenger, and a protein that may be deficient or downregulated during development of cardiomyopathy, and a biologically active derivative thereof, to treat a coronary indication.

Another embodiment is is use of a polypeptide therapeutic agent including an inhibitor of interleukin 1β converting enzyme, tissue plasminogen activator (tPA), urokinase plasminogen activator (uPA), urokinase, streptokinase, an inhibitor of α2 plasmin inhibitor, an inhibitor of plasminogen activator inhibitor-1 (PAI-1), basic fibroblast growth factor (bFGF), acidic fibroblast growth factor (aFGF), vascular endothelial cell growth factor (VEGF), angiogenin, transforming growth factor α (TGF-α), transforming growth factor β (TGF-β), tumor necrosis factor-α (TNF-α), platelet derived growth factor (PDGF), placental growth factor (PGF), hepatocyte growth factor, proliferin, decay accelerating factor, CAB-2, tissue factor pathway inhibitor (TFPI), heparin, hirudin, protein C, protein S, anti-thrombin III, tick anti-coagulant peptide (TAP), anti-stasin, glycoprotein IIb/IIa antagonist, antibodies, Herpes thymidine kinase, fas, faf, platelet factor 4, thrombospondin, a tissue inhibitor of a metalloproteinase, prolactin, bFGF soluble receptor, a proliferin-related protein, myocyte growth factor, superoxide dismutase (SOD), troponin C, beta-adrenergic receptor, insulin-like growth factor I (IGF-I), nematode anti-coagulant protein (NAP), biologically active fragments thereof, and chimeras thereof, to treat a coronary condition.

In accordance to a further object the polynucleotide administered encodes L-aromatic amino acid decarboxylase. In accordance with another object a second therapeutic agent can be administered including L-Dopa, tyrosine, a polynucleotide encoding tyrosine hydroxylase, or a tyrosine hydroxylase polypeptide.

Another embodiment of the invention is a method of targeting a specific area of the myocardium by identifying a region to target, and administering to the pericardial space in the target region a viscous pharmaceutical composition comprising a therapeutic agent. Further objects, features, and advantages of the present invention will become apparent from the following detailed description. It should be understood, however, that the detailed description, while indicating preferred embodiments of the present invention, is given by way of illustration only, since various changes and modifications within the spirit and scope of the invention will become apparent to those skilled in the art from this detailed description. The invention is also not limited to any theories of action of the elements of the invention.

### Detailed Description

The invention described herein draws on previously published work and pending patent applications. By way of example, such work consists of scientific papers, patents or pending patent applications.

### Definitions

The term "coronary condition" refers to" to a diagnosis or presumptive diagnosis of cardiovascular disease or conditions affecting the heart that are associated with atherosclerosis, ischemic syndromes, cardiomyopathies, antiythmias, dyserhythmias, hypertension and infections. The diagnosis can be made based on pain, fatigability, weakness, palpitations, and systemic symptoms that may be due to the cardiac disease or that may accompany it. Determination of a cardiovascular indication may include a physical exam and other non-invasive diagnostic procedures including radionuclide imaging, positron emission tomography, magnetic resonance imaging, echocardiography, and can also include venous and arterial cannulation and pulmonary and cardiac catheterization used in diagnosis of the cardiac condition. The term "cardiovascular indication" refers to a condition that affects the heart, and generally implies a coronary condition.

The term "treatment" as used herein refers to reducing or alleviating symptoms in a subject, preventing symptoms from worsening or progressing, inhibition or elimination of the causative agent, or prevention of the infection or disorder in a subject who is free therefrom. Thus, for example, treatment of a cardiovascular condition in a patient may be reduction of symptoms of heart failure or disfunction, such as reduction of angina, irregular heart beat, or other symptoms of cardiovascular impairment.

The term "prevention" refers to a prophylactic administration in a patient in whom it is expected a cardiovascular condition may develop. A preventative administration might occur before, for example surgery, or might be appropriate where a person has suffered a mild heart attack, and it is feared that a more severe heart attack is likely without some palliative or preventative treatment. A preventative treatment would aim to prevent a harm from occurring to the heart muscle and surrounding tissue, and persons to whom such an administration is most appropriate are persons who are likely candidates for such harm based on other contributing factors or symptoms.

The term "administering to the pericardial space" or "administering intrapericardially" as used herein refers to any method of administration that effects delivery of a therapeutic agent into the pericardial space. The pericardial space may be the entire region comprising the pericardial space, or only a part of it. The term "administering into the pericardial space" is synonymous with the terms "intrapericardial delivery" and "pericardial delivery", and can include delivery to subregions of the pericardial space that form interfaces between the pericardial space and the tissue that surrounds and forms it. The administration into the pericardial space can be accomplished by, for example, the following means of administration including injection, laser, catheter, pump. Intrapericardial delivery of the polynucleotides and the drugs of the invention can be accomplished by the methods of such delivery as disclosed in, for example, U.S. Patent Nos. 5,137,510, 5,269,326, and 5,213,570, herein incorporated by reference. Administration to the pericardial space can be by internal or external means.

"Internal entry" into the pericardial space refers to administration through an atrium or ventricle of the heart, for example the left atrium or the right or left ventricle.

"External entry" into the pericardial space refers to administration through the skin in the front of the chest and into the pericardial space via the chest cavity. This external administration can be facitilated by minimally invasive surgery (MIS), open chest procedure, or percutaneous entry. The percutaneous entry can be accomplished by using a device such as, for example, a needle, a catheter, a cannular, or a trocar.

The term "therapeutically effective amount" as used herein refers to an amount of a therapeutic agent to treat or prevent a cardiovascular condition sufficient to exhibit a detectable therapeutic or preventative effect. The effect may include, for example, treatment or prevention of the cardiovascular conditions listed herein. The precise effective amount for a subject will depend upon the subject's size and health, the nature and extent of the cardiovascular condition, and the therapeutics or combination of therapeutics selected for administration. Thus, it is not useful to specify an exact effective amount in advance. However, the effective amount for a given situation can be determined by routine experimentation.

The term "therapeutic agent" as used herein encompasses prophylactic agents and refers to any drugs, genes, proteins, polypeptides, peptides and peptoids, or other small molecules and in general, any molecular agent that may be useful to treat or prevent a cardiovascular condition. A therapeutic agent is selected based on the diagnosis of the patient and the particular goals of the therapy. Therapeutic agents for treatment of cardiovascular conditions are numerous and well known, and include but are not limited to the therapeutic agents listed herein. Any therapeutic agent of the invention may be administered with a pharmaceutically acceptable carrier.

The term "pharmaceutically acceptable carrier" refers to a carrier for administration of a therapeutic agent, such as antibodies or a polypeptide, drugs as listed herein, genes, and other therapeutic agents listed herein, *in vivo*, and refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in REMINGTON'S PHARMACEUTICAL SCIENCES (Mack Pub. Co., N.J. 1991). Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

The term "liposomes" refers to, for example, the liposome compositions described in U.S. Patent No. 5,422,120, WO 95/13796, WO 94/23697, WO 91/14445 and EP 524,968 B1. Liposomes may be pharmaceutical carriers for the drugs or polynucleotides or the combination of the two.

The term "drug" as used herein refers to any number of therapeutic molecules, many of which are disclosed herein, that are used for treatment or prevention of cardiovascular conditions. A drug can be a small organic molecule, a peptide, a peptoid, a polynucleotide or a polypeptide, for example. Drugs useful herein can include an anti-apoptotic agent, a thrombolytic agent, a pro-angiogenic agent, an anti-arrythmic agent, a contractility improving agent, a complement blocker, an inhibitor of reperfusion injury, a calcium channel blocker, a beta-blocker, an afterload reducer, a preload reducer, a vasoactive agent, an anti-thrombotic agent, an anti-platelet agent, anti-proliferative agent, an anti-inflammatory agent, an immunomodulating agent, an immunosuppressive agent, an inhibitor of reactive oxygen metabolites, an anti-angiogenic agent, a myocyte growth factor, a vasoactive agent, a cardioprotective agent, an iron-chelating agent, an anti-hypertensive agent, an anti-integrin agent, a pro-apoptotic agent, an anti-viral agent, an anti-parasitic agent, a free radical scavenger, and a protein that may be deficient or downregulated during development of cardiomyopathy, a biologically active fragment thereof and a chimera thereof.

The term "matrices" refers to compositions containing polymers, natural or synthetic, including gels, including biodegradable gels, collagens, gelatin, and fibrinogen.

The term "polynucleotide" as used herein refers to a nucleic acid molecule or a coding sequence that encodes a specific amino acid sequence or its complementary strand. The polynucleotide, nucleic acid molecule or coding sequence can be either DNA or RNA. The DNA molecule can be genomic DNA, or cDNA. The nucleic acid molecule can be naturally occurring or synthetically made. The nucleic acid molecule can be under control of a regulatory sequence.

A "regulatory sequence" refers to a nucleic acid sequence encoding one or more elements that are capable of affecting or effecting expression of a gene sequence, including transcription or translation thereof, when the gene sequence is placed in such a position as to subject it to the control thereof. Such a regulatory sequence can be, for example, a minimal promoter sequence, a complete promoter sequence, an enhancer sequence, an upstream activation sequence ("UAS"), an operator sequence, a downstream termination sequence, a polyadenylation sequence, an optimal 5' leader sequence to optimize initiation of translation, and a Shine-Dalgarno sequence. Alternatively, the regulatory sequence can contain a combination enhancer/promoter element. The regulatory sequence that is appropriate for expression differs depending upon the host system used for expression. Selection of the appropriate regulatory sequences for use herein is within the capability of one skilled in the art. For example, in prokaryotes, such a regulatory sequence can include one or more of a promoter sequence, a ribosomal binding site, and a transcription termination sequence. In eukaryotes, for example, such a sequence can include one or more of a promoter sequence and/or a transcription termination sequence. Regulatory sequences suitable for use herein may be derived from any source including a prokaryotic source, an eukaryotic source, a virus, a viral vector, a bacteriophage or a linear or circular plasmid. Alternatively, the regulatory sequence herein can be a synthetic sequence such as, for example, one made by combining the UAS of one gene with the remainder of a requisite promoter from another gene, such as the GADP/ADH2 hybrid promoter.

The term "combination" as used herein refers to a combination or mixture of at least one polynucleotide and at least one drug in the same or separate pharmaceutical compositions for administration to the pericardial space according to the invention. A combination is warranted for those cardiovascular indications that respond to both the drug and the polynucleotide when administered together to effect treatment of the cardiovascular condition. The administration of the combination may be simultaneous, or consecutive, in close proximity in time relative to the responsiveness of each therapeutic. Thus, where a polynucleotide is transformed into the cells of the pericardial tissue within a week of administration into the pericardial space, administration into the pericardial space of a companion drug may occur, for example, up to one week after the initial administration of the polynucleotide and still be considered to have been administered as a combination.

The term "biologically active fragment" refers to fragments of protein or polypeptides that retain one or more of the activities of the full-length protein. In referenced to a protein or polypeptide in the context of the therapeutics and preventatives of the invention and the gene products produced by gene therapy according to the invention, it is contemplated that such protein or polypeptides include biologically active fragments, truncations, variants, alleles, analogs and derivatives thereof. Unless specifically mentioned otherwise, such truncations, variants, alleles, analogs, and derivatives possess one or more of the bioactivities of the native mature protein. This term is not limited to a specific length of the product of the gene. Thus, polypeptides that are identical or contain at least 60%, preferably 70%, more preferably 80%, and most preferably 90% homology to the native protein fragment or the native mature protein, wherever derived, from human or nonhuman sources are included within the term polypeptide. The term polypeptide also does not exclude post-expression modifications of the polypeptide, for example, glycosylations, acetylations, phosphorylations and the like.

"Alleles" and "variants" refers to a polypeptide that differs from the native specified protein by virtue of one or more amino acid substitutions, deletions, or insertions. The amino acid substitutions can be conservative amino acid substitutions or substitutions to eliminate non-essential amino acid residues such as to alter a glycosylation site, a phosphorylation site, an acetylation site, or to alter the folding pattern by altering the position of the cysteine residue that is not necessary for function, etc. Conservative amino acid substitutions are those that preserve the general charge, hydrophobicity/hydrophilicity and/or steric bulk of the amino acid substituted, for example, substitutions between the members of the following groups are conservative substitutions: Gly/Ala, Val/Ile/Leu, Asp/Glu, Lys/Arg, Asn/Gln, Ser/Cys/Met and Phe/Trp/Tyr.

"Analogs" include peptides having one or more peptide mimics, also known as peptoids, that possess the activity sought. Included within the definition are, for example, polypeptides containing one or more analogs of an amino acid, including, for example, unnatural amino acids, etc., polypeptides with substituted linkages, as well as other modifications known in the art, both naturally occurring and non-naturally occurring. An analog of a polypeptide or other molecule with bioactivity is generally considered to be an agonist of the original actor, and thus able to generate the same or similar bioactivity as the parent molecule that it mimics.

The term "chimera" as used herein in reference to polynucleotide or a polypeptide denotes a fusion molecule of the coding regions or expression products or portions thereof, respectively, where the coding regions or expression products are not normally contiguous.

A "nucleic acid molecule" or a "polynucleotide," as used herein, refers to either RNA or DNA molecule that encodes a specific amino acid sequence or its complementary strand. Nucleic acid molecules may also be non-coding sequences, for example, a ribozyme, an antisense oligonucleotide, or an untranslated portion of a gene. A "coding sequence" as used herein, refers to either RNA or DNA that encodes a specific amino acid sequence or its complementary strand. A polynucleotide may include, for example, an antisense oligonucleotide, or a ribozyme, and may also include such items as a 3' or 5' untranslated region of a gene, or an intron of a gene, or other region of a gene that does not make up the coding region of the gene. The DNA or RNA may be single stranded or double stranded. Synthetic nucleic acids or synthetic polynucleotides can be chemically synthesized nucleic acid sequences, and may also be modified with chemical moieties to render the molecule resistant to degradation. Synthetic nucleic acids can be ribozymes or antisense molecules, for example. Modifications to synthetic nucleic acid molecules include nucleic acid monomers or derivative or modifications thereof, including chemical moieties. For example, phosphothioates can be used for the modification. A polynucleotide derivative can include, for example, such polynucleotides as branched DNA (bDNA). A polynucleotide can be a synthetic or recombinant polynucleotide, and can be generated, for example, by polymerase chain reaction (PCR) amplification, or recombinant expression of complementary DNA or RNA, or by chemical synthesis.

"Coronary artery occlusion" refers to occlusion or reocclusion of a coronary artery as occurs in atherosclerosis, thrombosis, and restenosis.

A "combination therapeutic agent" is a therapeutic composition having several components that produce when administered together their separate effects. The separate effects of the combination therapeutic agent combine to result in a larger therapeutic effect, for example improved prognosis with a cardiovascular condition. An example of separate effects resulting from administration of a combination therapeutic agent is the combination of such effects as a pro-angiogenic effect and an anti-apoptotic effect. The combination therapeutic agent is more than one therapeutic agent delivered in the same pharmaceutical composition in the same administration.

The inventor has discovered that pericardial delivery using gene therapy may be applied to the treatment of cardiovascular conditions since the constitutive or inducible expression of therapeutic gene products in the heart may obviate the need for repeated administration of therapeutic agents or reduce the stringency of requirements for high capacity, prolonged release, long-acting, extremely stable drug formulations. Furthermore, the inducible expression of therapeutic gene products has the advantage of being relatively easily regulatable. Because of the non-proliferative and terminally differentiated state of cardiomyocytes, myocardial somatic gene therapy must employ *in vivo* gene-transfer technologies using vectors and delivery systems that can transduce non-proliferating cells. Previously, delivery of genes to the heart has been accomplished by direct injection of genes encoded in both viral and non-viral vectors to the myocardium. Some of the difficulties with direct myocardial injection include the logisitical problems of myocardial trauma and its attendant effects such as myocardial necrosis, fibrosis, inflammation, arrhythmia's, and bleeding problems with delivery of genes to a large myocardial surface area, and the practical limitations of this technique for sustained or repeated administrations.

Barr *et al., Gene Therapy* (1994) *1*:51-58 describes gene delivery via catheter-mediated infusion of replication defective adenovirus into the coronary arterial circulation. High level expression of exogenous gene was obtained throughout the thickness of the ventricular and arterial walls within the distribution of the injected coronary artery. However, infection of multiple cell types and non-cardiac tissues and the binding of adenovirus DNA in the brain and testis raised safety concerns.

The inventor herein has discovered that delivery of therapeutic agents including polynucleotides and drugs to the pericardial space results in higher, more prolonged, and more effective drug delivery to arterial structures than local intra-arterial drug delivery. Deposition of agents within the pericardium is a method of achieving prolonged, high concentration, high capacity drug delivery to the coronary artery.

More prolonged and higher concentration drug delivery is possible from the pericardium as compared to intracoronary delivery because the storage capacity of the pericardium for therapeutic agents is significantly larger than that of the coronary vessel and the agents deposited in the pericardium are not subject directly to flowing blood as are agents deposited directly into coronary artery vasculature. Due to the diffusion of intrapericardially delivered agents across the coronary vessel adventitia into the coronary vascular lumen and, therefore, the coronary artery circulation, deposition within the pericardium is an effective method of achieving prolonged, high concentration, high capacity delivery of therapeutic agents to the myocardium, endocardium, or other designated cardiac targets while minimizing systemic exposure and therefore toxicity.

The invention achieves a reduction in systemic toxicity. Systemic toxicity is often experienced with other methods of treating cardiovascular conditions. Because the pericardial sack is a natural closed space the systemic toxicity of agents can be locally contained in this closed space by the method of the invention, and systemic toxicity that is largely influenced and regulatable by the access of the agents to the coronary and myocardial vasculature is controlled.

The invention facilitates access of intrapericardially delivered agents to the myocardium or endocardium by formulations of agents that improve the agents' ability to penetrate deeply into tissues or to penetrate the coronary vessel adventitia, to access the coronary vessel lumen and, therefore, enter the coronary circulation. Also, according to the invention, the creation of either natural or artificial conduits between the pericardial space and the myocardium or endocardium, improves access of intrapericardial agents to these areas of the heart. An example of a method of creating "natural" conduits between the pericardial space and myocardium is the use of proangiogenic factors delivered into the pericardial space to increase the vascularization of the myocardium globally or in a specific area and, therefore, increase the accessibility of agents in the pericardium to these areas. An example of a method of creating an "artificial" conduit between the pericardial space and myocardium or endocardium is the use of laser, as is currently being used in myocardial revascularization, to create direct channels between these structures to increase the accessibility of agents in the pericardium to deeper myocardial regions.

The inventor has also discovered that delivery of genes to the intrapericardial space is a safer and more effective method of accomplishing myocardial gene therapy. According to the method of the invention, delivery of genes to the pericardial space does not require mechanical violation of the myocardium as does direct myocardial injection. Secondly, because intrapericardially delivered agents have access to the entire myocardial surface the ease and effectiveness with which genes can be delivered to large areas of myocardium is increased. When these agents also, in turn, access the coronary circulation, perfusion of the entire heart with these agents occurs. Third, the inventor has found that the pericardium is more easily transducible than myocardium and, thus, that expression of gene products in the pericardial space retains access to myocardium. The method of administration of the invention is, thus, preferable to previous methods that have attempted expression of gene products in the myocardium with limited success.

The inventor has also found that the exposure time of nucleic acids and/or viruses to cells, which is an important determinant of transduction or infection efficiency, increases in the invention. Genetic agents deposited in the pericardial space are not subject to rapid dilution, drainage, or dissipation due to blood flow or lymphatic clearance, and thus have much longer exposure times than vascularly delivered agents, also increasing the transduction of infection efficiency of the genes. Such an advantage translates into much higher transduction or infection efficiency with genes and/or viruses in either the myocardium or the pericardium than is achievable in the coronary vessel. Lastly, because pericardium is highly efficient at expressing certain proteins and in some cases is even more efficient than myocardium at this task, the method of the invention is a new and improved method of delivery of genes for gene therapy for treatment of a cardiovascular indication.

Practice of the invention also includes, for example, delivering into the pericardial space cardiovascular therapeutics, whether genes or drugs, in liposomal compositions, including heterovesicular liposomes. Delivery in liposomes increases the efficacy of the cardiovascular therapeutic, and reduces the dosage requirements and, in general, augments the benefits of any cardiovascular therapeutic delivered into the pericardial space. Suitable liposomal formats include, for example, the liposome compositions described in U.S. Patent No. 5,422,120, WO 95/13796, WO 94/23697, WO 91/14445 and EP 524,968 B1. Liposomes may be pharmaceutical carriers for the small molecules, polypeptides or polynucleotides of the invention, or for combination of these therapeutics. Liposomes are included within the definition of a pharmaceutically acceptable carrier. Polypeptide therapeutics can also be delivered with the gene that is delivered for expression in the patient, for example, before, with, or after the gene delivery, and polypeptide therapeutic agents can be delivered with any cardiac drugs that are also delivered before, with or after the gene delivery.

Delivery to the pericardial space of any therapeutic agent, including proteins, polypeptide, polynucleotides, or other drugs can be accomplished by internal entry via access of the right-sided circulation (right heart catheterization with entry through the atrium or ventricle), left-sided circulation (left heart catheterization, as can occur during balloon angioplasty, with entry through the atrium or ventricle). Alternatively, access to the pericardial space can be made by an external approach, which can include open chest procedures, a "minimally invasive surgery" (MIS) procedure, or percutaneous approach using a needle, catheter, cannula, trocar, or other pericardial access device. Preferred agents for such administration include any of polypeptide, polynucleotide or other drug, including but not limited to growth factors, for example, fibroblast growth factors (including mammalian bFGF, aFGF, FGF-5) and IGF-1, and any pro-angiogenic or cardiotrophic factors.

With regard to internal entry into the pericardial space, entry through the ventricle may prove to be safer that entry through the atrium, since the ventricle is a more muscular and thicker walled structure than the atrium. The greater thickness of cardiac muscle in the ventricle, and the greater contractility of ventricular (as opposed to atrial) muscle, potentially allow an entry tract from the ventricle to the pericardial space to be more efficiently "resealed" than a similar tract through an atrial wall. The greater thickness of the ventricular wall (as compared to the atrial wall) also diminishes the chance for blood to leak across the myocardium to the pericardial space and cause hemopericardium or pericardial tamponade.

Additionally, because the pericardium is a sac surrounding the heart, any sufficiently fluid composition deposited in the pericardium will have access to all pericardial surfaces depending on the volume of fluid in the pericardial space and the position of the subject. The invention, however, is not limited by any theories of mechanism. In some coronary conditions, adhesions between the pericardium and epicardial surface of the heart can occur. These adhesions can be due to many processes, some of which are inflammatory, infectious, malignant, or ischemic. Such adhesions may limit the ability of an intrapericardially deposited fluid composition to access all epicardial surfaces. If widespread epicardial access is desired but limited by pericardial adhesions, a potential solution is to deposit agents capable of lysing pericardial/epicardial adhesions (such as fibrinolytic agents like TPA, streptokinase or urokinase, or collagenases or matrix metalloproteinases), in order to increase the access of therapeutic agents in the pericardium to the entire heart surface. The therapeutic agents for lysing pericardial/epicardial adhesions can be delivered in a viscous pharmaceutical composition, for example a gel or matrix, including also, for example, a biodegradable gel, with the administration, for example an external administration, targeted to the region of the pericardial space where lesions are detected. This method of administration can localize the therapeutic agent for action in the region of greatest efficacy. Thus a method of targeting a specific area of the myocardium can be accomplished by identifying a region to target, and administering to the pericardial space in the target region a viscous, adherent, or otherwise localizable pharmaceutical composition having a therapeutic agent. Furthermore, mechanical means such as insuflation of air or infusion of liquid into the pericardium might be employed alone, or in conjunction with agents capable of lysing pericardial/epicardial adhesions, in order to increase the access of intrapericardially delivered agents to the entire surface of the heart.

In some coronary conditions, exposure of the entire surface of the heart to an intrapericardially delivered therapeutic agent may not be desirable. As an example, although bFGF can improve myocardial ischemia by causing myocardial revascularization, bFGF is also a smooth muscle cell mitogen, in addition to being an endothelial cell mitogen. Although the invention is not limited to theories of mechanism, some evidence suggests that the stenosis of atherosclerotic, traumatized, or otherwise diseased coronary arteries might be exacerbated by a growth factor capable of inducing vascular smooth cell proliferation.

Therefore, for treatment of particular coronary conditions, targeting an intrapericardially delivered therapeutic agent, for example, bFGF polypeptide, specifically to an area of myocardium requiring revascularization (i.e. ischemic myocardium at risk for infarction), but not to the entire myocardial surface (i.e. areas of myocardium supplied by significantly but not critically stenotic coronary vessels) can be accomplished by a number of means including but not limited to, for example, delivering an adherent gel, a polymer, or other substance impregnated with the therapeutic agent that can be delivered intrapericardially. That delivery is then targeted to only to the desired epicardial surface.

Thus, a method of treating a cardiac muscle tissue can be designed by first identifying an infarct or ischemic zone, for example, by some form of imaging, accessing the pericardial space in the region of the infarct or ischemic zone by, for example an external entry, and delivering a pharmaceutical composition having a therapeutic agent to the region of the infarct or ischemic zone. The therapeutic agent can be in a pharmaceutical composition, for example a gel or matrix. As an example, a cardiac imaging procedure (i.e. thallium scan or EKG) can be used to identify a region of myocardium in need of medical revascularization. The patient can be positioned so that the area of myocardium is in a dependent position. A gel that polymerizes at body temperature, impregnated with a therapeutic compound (i.e. bFGF) can be delivered to the pericardium so that it polymerizes on the epicardial surface overlying the area of myocardium requiring revascularization.

Drugs delivered to the pericardium can also be targeted to specific areas of myocardium (i.e. ischemic or infarcted areas) by linking them to targeting agents (i.e. antibodies or ligands) which bind targets (i.e. cell surface molecules such as, for example, integrins, or cell surface receptors) that are upregulated or overexpressed in the diseased areas of myocardium by virtue of the underlying disease process (i.e. myocardial cell injury). Drugs that become active only under certain conditions (i.e. low pH) that arise in diseased myocardial areas (i.e. ischemic zones) might also be a means of increasing the specificity of an intrapericardially delivered therapeutic agent for diseased areas of myocardium.

Intrapericardial delivery targeting regions of the pericardial space, and thus targeting specific diseased areas of myocardium, can be accomplished by intrapericardially deliverying therapeutic agents via a right heart catheterization or a left heart catheterization. Therapeutic agents can also be delivered through external access of the pericardium into the pericardial space through the chest cavity, for example to regions of the pericardial space proximal to areas of the myocardium that indicate, for example, myocardial infarcts or ischemia.

In all cases of treatment of coronary conditions, the therapeutic agent can be an anti-apoptotic agent, a thrombolytic agent, a pro-angiogenic agent, an anti-arrythmic agent, a contractility improving agent, a complement blocker, an inhibitor of reperfusion injury, a calcium channel blocker, a beta-blocker, an afterload reducer, a preload reducer, a vasoactive agent, an anti-thrombotic agent, an anti-platelet agent, anti-proliferative agent, an anti-inflammatory agent, an immunomodulating agent, an immunosuppressive agent, an inhibitor of reactive oxygen metabolites, an anti-angiogenic agent, a myocyte growth factor, a vasoactive agent, a cardioprotective agent, an iron-chelating agent, an anti-hypertensive agent, an anti-integrin agent, a pro-apoptotic agent, an anti-viral agent, an anti-parasitic agent, a free radical scavenger, an anti-tumor agent, and a protein that may be deficient or downregulated during development of cardiomyopathy, or biologically active derivatives thereof.

In all cases of intrapericardial delivery the therapeutic agent can be a polypeptide tissue plasminogen activator (tPA), an inhibitor of interleukin 1β converting enzyme, urokinase plasminogen activator (uPA), urokinase, streptokinase, an inhibitor of α2 plasmin inhibitor, an inhibitor of plasminogen activator inhibitor-1 (PAI-1), basic fibroblast growth factor (bFGF), acidic fibroblast growth factor (aFGF), vascular endothelial cell growth factor (VEGF), angiogenin, transforming growth factor α (TGF-α), transforming growth factor β (TGF-β), tumor necrosis factor-α (TNF-α), platelet derived growth factor (PDGF), placental growth factor (PGF), hepatocyte growth factor, proliferin, decay accelerating factor, CAB-2, tissue factor pathway inhibitor (TFPI), heparin, hirudin, protein C, protein S, anti-thrombin III, tick anti-coagulant peptide (TAP), anti-stasin, glycoprotein IIb/IIa antagonist, antibodies, Herpes thymidine kinase, fas, faf, platelet factor 4, thrombospondin, a tissue inhibitor of a metalloproteinase, prolactin, bFGF soluble receptor, a proliferin-related protein, myocyte growth factor, superoxide dismutase (SOD), troponin C, beta-adrenergic receptor, insulin-like growth factor I (IGF-I), nematode anti-coagulant protein (NAP), biologically active fragments thereof, or chimeras thereof. NAP and other thrombolytic agents are described in WO 96/12021, incorporated by reference in full.

The pharmaceutical composition can have a combination of more than one therapeutic agent. For example, two polypeptides, or a small organic molecule and a polypeptide can be placed in the same pharmaceutical composition for delivery. Thus, for example, bFGF and IGF-1 can be co-administered in the same pharmaceutical composition.

Additionally, the heart can be more completely accessed by an administration of a therapeutic agent to a pericardial space by administering an agent capable of lysing a pericardial/epicardial adhesion, alone, or by also expanding the pericardial space. The agent can be, for example, a fibrinolytic agent, tissue plasminogen activator (tPA), streptokinase, urokinase, collagenase, and a matrix metalloprotease. The pericardial space expansion can be accomplished by placing a liquid or air into the space, and then administering an agent. This expansion can be temporary, with the purpose of releasing the binding of the epicardium to the myocardium. The following expression systems detail promoters and vectors useful for gene therapy applications of the invention for the intrapericardial delivery of polynucleotides in plasmids, viral vectors or liposomes, or for the production of polypeptides that are delivered intrapericardially.

Although the methodology described below is believed to contain sufficient details to enable one skilled in the art to practice the present invention, other items not specifically exemplified, such as plasmids, can be constructed and purified using standard recombinant DNA techniques described in, for example, Sambrook *et al*. (1989), MOLECULAR CLONING: A LABORATORY MANUAL, 2d edition (Cold Spring Harbor Press, Cold Spring Harbor, N.Y.), and Ausubel *et al*., CURRENT PROTOCOLS IN MOLECULAR BIOLOGY (1994), (Greene Publishing Associates and John Wiley & Sons, New York, N.Y.). under the current regulations described in United States Dept. of HEW, NATIONAL INSTITUTE OF HEALTH (N1H) GUIDELINES FOR RECOMBINANT DNA RESEARCH. These references include procedures for the following standard methods: cloning procedures with plasmids, transformation of host cells, cell culture, plasmid DNA purification, phenol extraction of DNA, ethanol precipitation of DNA, agarose gel electrophoresis, purification of DNA fragments from agarose gels, and restriction endonuclease and other DNA-modifying enzyme reactions.

### Expression in Bacterial Cells

The polynucleotide of the present invention can be produced in prokaryotes, for example bacteria. Further, the proteins and polypeptide drugs of the present invention can also be produced recombinantly in prokaryotes. Control elements for use in bacteria include promoters, optionally containing operator sequences, and ribosome binding sites. Useful promoters include sequences derived from sugar metabolizing enzymes, such as galactose, lactose (*lac*) and maltose. Additional examples include promoter sequences derived from biosynthetic enzymes such as tryptophan (*trp*), the β-lactamase (*bla*) promoter system, bacteriophage λPL, and T7. In addition, synthetic promoters can be used, such as the *tac* promoter. The β-lactamase and lactose promoter systems are described in Chang *et al., Nature* (1978) 275: 615, and Goeddel *et al., Nature* (1979) *281*:544; the alkaline phosphatase, tryptophan (trp) promoter system are described in Goeddel *et al., Nucleic Acids Res.* (1980) *8*: 4057 and EP 36,776 and hybrid promoters such as the *tac* promoter is described in U.S. Patent No. 4,551,433 and deBoer *et al., Proc. Natl. Acad*. *Sci. USA* (1983) *80*: 21-25. However, other known bacterial promoters useful for expression of eukaryotic proteins are also suitable. A person skilled in the art would be able to operably ligate such promoters to the coding sequences of interest, for example, as described in Siebenlist *et al., Cell* (1980) *20*: 269, using linkers or adaptors to supply any required restriction sites. Promoters for use in bacterial systems also generally will contain a Shine-Dalgarno (SD) sequence operably linked to the DNA encoding the target polypeptide. For prokaryotic host cells that do not recognize and process the native target polypeptide signal sequence, the signal sequence can be substituted by a prokaryotic signal sequence selected, for example, from the group of the alkaline phosphatase, penicillinase, Ipp, or heat stable enterotoxin II leaders. The origin of replication from the plasmid pBR322 is suitable for most Gram-negative bacteria.

The foregoing systems are particularly compatible with *Escherichia coli.* However, numerous other systems for use in bacterial hosts including Gram-negative or Gram-positive organisms such as *Bacillus spp., Streptococcus spp., Streptomyces spp., Pseudomonas* species such as *P. aeruginosa, Salmonella typhimurium*, or *Serratia marcescans,* among others. Methods for introducing exogenous DNA into these hosts typically include the use of CaCl₂ or other agents, such as divalent cations and DMSO. DNA can also be introduced into bacterial cells by electroporation, nuclear injection, or protoplast fusion as described generally in Sambrook *et al*. (1989), cited above. These examples are illustrative rather than limiting. Preferably, the host cell should secrete minimal amounts of proteolytic enzymes. Alternatively, *in vitro* methods of cloning, e.g., PCR or other nucleic acid polymerase reactions, are suitable.

Prokaryotic cells used to produce the target polypeptide of this invention are cultured in suitable media, as described generally in Sambrook *et al*., cited above.

### Expression in Yeast Cells

The polynucleotide, proteins and polypeptides and polypeptides of the present invention can also be produced in eukaryotic systems including, for example, yeast cells, insect cells, and mammalian cells. Expression and transformation vectors, either extrachromosomal replicons or integrating vectors, have been developed for transformation into many yeasts. For example, expression vectors have been developed for, among others, the following yeasts: *Saccharomyces cerevisiae* ,as described in Hinnen *et al., Proc. Natl. Acad. Sci. USA* (1978) *75*: 1929; Ito *et al., J. Bacterial.* (1983) *153*: 163; *Candida albicans* as described in Kurtz *et al., Mol. Cell. Biol.* (1986) *6*: 142; *Candida maltosa,* as described in Kunze *et al., J. Basic Microbiol.* (1985) *25*: 141; *Hansenula polymorpha*, as described in Gleeson *et al., J. Gen. Microbiol.* (1986) *132*: 3459 and Roggenkamp *et al., Mol. Gen. Genet.* (1986) *20*2 :302); *Kluyveromyces fragilis,* as described in Das *et al., J. Bacteriol.* (1984) *158*: 1165; *Kluyveromyces lactis*, as described in De Louvencourt *et al., J. Bacteriol.* (1983) *154*: 737 and Van den Berg *et al., Bio*/*Technology* (1990) *8*: 135; *Pichia guillerimondii*, as described in Kunze *et al., J. Basic Microbiol.* (1985) *25*: 141; *Pichia pastoris*, as described in Cregg *et al., Mol. Cell. Biol.* (1985) *5*: 3376 and U.S. Patent Nos. 4,837,148 and 4,929,555; *Schizosaccharomyces pombe,* as described in Beach and Nurse, *Nature* (1981) *300*: 706; and *Yarrowia lipolytica*, as described in Davidow *et al., Curr. Genet.* (1985) *10*: 380 and Gaillardin *et al., Curr. Genet.* (1985) *10*: 49, *Aspergillus* hosts such as A. *nidulans*, as described in Ballance *et al., Biochem. Biophys. Res. Commun.* (1983) *112*: 284-289; Tilburn *et al., Gene* (1983) *26*: 205-221 and Yelton *et al., Proc. Natl. Acad. Sci. USA* (1984) *81*: 1470-1474, and *A. niger,* as described in Kelly and Hynes, *EMBO J.* (1985) *4*: 475479; *Trichoderma reesia*, as described in EP 244,234, and filamentous fungi such as, e.g, *Neurospora, Penicillium, Tolypocladium*, as described in WO 91/00357.

Control sequences for yeast vectors are known and include promoters regions from genes such as alcohol dehydrogenase (ADH), as described in EP 284,044, enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK), as described in EP 329,203. The yeast *PHO5* gene, encoding acid phosphatase, also provides useful promoter sequences, as described in Myanohara *et al., Proc. Natl. Acad. Sci. USA* (1983) *80*: 1. Other suitable promoter sequences for use with yeast hosts include the promoters for 3-phosphoglycerate kinase, as described in Hitzeman *et al., J. Biol. Chem.* (1980) *255*: 2073, or other glycolytic enzymes, such as pyruvate decarboxylase, triosephosphate isomerase, and phosphoglucose isomerase, as described in Hess *et al., J. Adv. Enzyme Reg.* (1968) *7*:149 and Holland *et al., Biochemistry* (1978) *17*:4900. Inducible yeast promoters having the additional advantage of transcription controlled by growth conditions, include from the list above and others the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in Hitzeman, EP 073,657. Yeast enhancers also are advantageously used with yeast promoters. In addition, synthetic promoters which do not occur in nature also function as yeast promoters. For example, upstream activating sequences (UAS) of one yeast promoter may be joined with the transcription activation region of another yeast promoter, creating a synthetic hybrid promoter. Examples of such hybrid promoters include the ADH regulatory sequence linked to the GAP transcription activation region, as described in U.S. Patent Nos. 4,876,197 and 4,880,734. Other examples of hybrid promoters include promoters which consist of the regulatory sequences of either the *ADH2, GAL4, GAL10,* or *PHO5* genes, combined with the transcriptional activation region of a glycolytic enzyme gene such as *GAP* or *PyK*, as described in EP 164,556. Furthermore, a yeast promoter can include naturally occurring promoters of non-yeast origin that have the ability to bind yeast RNA polymerase and initiate transcription.

Other control elements which may be included in the yeast expression vectors are terminators, for example, from *GAPDH* and from the enolase gene, as described in Holland *et al., J. Biol. Chem.* (1981) *256*: 1385, and leader sequences which encode signal sequences for secretion. DNA encoding suitable signal sequences can be derived from genes for secreted yeast proteins, such as the yeast invertase gene as described in EP 012,873 and JP 62,096,086 and the a-factor gene, as described in U.S. Patent Nos. 4,588,684, 4,546,083 and 4,870,008; EP 324,274; and WO 89/02463. Alternatively, leaders of non-yeast origin, such as an interferon leader, also provide for secretion in yeast, as described in EP 060,057.

Methods of introducing exogenous DNA into yeast hosts are well known in the art, and typically include either the transformation of spheroplasts or of intact yeast cells treated with alkali cations.

Transformations into yeast can be carried out according to the method described in Van Solingen *et al., J. Bact.* (1977) *130*:946 and Hsiao *et al., Proc. Natl. Acad. Sci. (USA)* (1979) *76*:3829. However, other methods for introducing DNA into cells such as by nuclear injection, electroporation, or protoplast fusion may also be used as described generally in Sambrook *et al*., cited above.

For yeast secretion the native target polypeptide signal sequence may be substituted by yeast signal sequences such as those derived from yeast invertase, α-factor, killer toxins, or acid phosphatase leaders. The origin of replication from the 2 µ plasmid origin is suitable for yeast. A suitable selection gene for use in yeast is the *trp*1 gene present in the yeast plasmid described in Kingsman *et al., Gene* (1979) *7*: 141 or Tschemper *et al., Gene* (1980) *10:*157. The *trp*1 gene provides a selection marker for a mutant strain of yeast lacking the ability to grow in tryptophan. Similarly, Leu2-deficient yeast strains (ATCC 20,622 or 38,626) are complemented by known plasmids bearing the *Leu2* Gene.

For intracellular production of the present polypeptides in yeast, a sequence encoding a yeast protein can be linked to a coding sequence of the polypeptide to produce a fusion protein that can be cleaved intracellularly by the yeast cells upon expression. An example, of such a yeast leader sequence is the yeast ubiquitin gene.

### Expression in Insect Cells

Baculovirus expression vectors (BEVs) are recombinant insect viruses in which the coding sequence for a foreign gene to be expressed is inserted behind a baculovirus promoter in place of a viral gene, e.g., polyhedrin, as described in Smith and Summers, U.S. Pat. No., 4,745,051.

An expression construct herein includes a DNA vector useful as an intermediate for the infection or transformation of an insect cell system, the vector generally containing DNA coding for a baculovirus transcriptional promoter, optionally but preferably, followed downstream by an insect signal DNA sequence capable of directing secretion of a desired protein, and a site for insertion of the foreign gene encoding the foreign protein, the signal DNA sequence and the foreign gene being placed under the transcriptional control of a baculovirus promoter, the foreign gene herein being the coding sequence of the polypeptide.

The promoter for use herein can be a baculovirus transcriptional promoter region derived from any of the over 500 baculoviruses generally infecting insects, such as, for example, the Orders Lepidoptera, Diptera, Orthoptera, Coleoptera and Hymenoptera including, for example, but not limited to the viral DNAs *of Autographo californica* MNPV, *Bombyx mori* NPV, *rrichoplusia ni* MNPV, *Rachlplusia ou* MNPV or *Galleria mellonella* MNPV, *Aedes aegypti, Drosophila melanogaster, Spodoptera frugiperda*, and *Trichoplusia ni.* Thus, the baculovirus transcriptional promoter can be, for example, a baculovirus immediate-early gene IEI or IEN promoter; an immediate-early gene in combination with a baculovirus delayed-early gene promoter region selected from the group consisting of a 39K and a *Hin*dIII fragment containing a delayed-early gene; or a baculovirus late gene promoter. The immediate-early or delayed-early promoters can be enhanced with transcriptional enhancer elements.

Particularly suitable for use herein is the strong polyhedrin promoter of the baculovirus, which directs a high level of expression of a DNA insert, as described in Friesen *et al*. (1986) "The Regulation of Baculovirus Gene Expression" in: THE MOLECULAR BIOLOGY OF BACULOVIRUSES (W.Doerfler, ed.); EP 127,839 and EP 155,476; and the promoter from the gene encoding the p10 protein, as described in Vlak *et al., J. Gen. Virol.* (1988) *69*:765-776.

The plasmid for use herein usually also contains the polyhedrin polyadenylation signal, as described in Miller *et al., Ann. Rev. Microbiol.* (1988) *42*:177 and a procaryotic ampicillin-resistance (*amp*) gene and an origin of replication for selection and propagation in *E. coli.* DNA encoding suitable signal sequences can also be included and is generally derived from genes for secreted insect or baculovirus proteins, such as the baculovirus polyhedrin gene, as described in Carbonell *et al., Gene* (1988) *73*:409, as well as mammalian signal sequences such as those derived from genes encoding human a-interferon as described in Maeda *et al., Nature* (1985) *315*:592-594; human gastrin-releasing peptide, as described in Lebacq-Verheyden *et al., Mol. Cell. Biol.* (1988) *8*: 3129; human IL-2, as described in Smith *et al., Proc. Natl. Acad. Sci. USA* (1985) *82*:8404; mouse IL-3, as described in Miyajima *et al., Gene* (1987) *58*:273; and human glucocerebrosidase, as described in Martin *et al., DNA* (1988) *7*:99.

Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* host cells have been identified and can be used herein. See, for example, the description in Luckow *et al., Bio*/*Technologvy*(1988) *6:* 47-55, Miller *et al*., in GENETIC ENGINEERING (Setlow, J.K. *et al*. eds.), Vol. 8 (Plenum Publishing, 1986), pp. 277-279, and Maeda *et al., Nature,* (1985) *315*: 592-594. A variety of such viral strains are publicly available, e.g., the L-1 variant of *Autographa californica* NPV and the Bm-5 strain of *Bombyx mori* NPV. Such viruses may be used as the virus for transfection of host cells such as *Spodoptera frugiperda* cells.

Other baculovirus genes in addition to the polyhedrin promoter may be employed to advantage in a baculovirus expression system. These include immediate-early (alpha), delayed-early (beta), late (gamma), or very late (delta), according to the phase of the viral infection during which they are expressed. The expression of these genes occurs sequentially, probably as the result of a "cascade" mechanism of transcriptional regulation. Thus, the immediate-early genes are expressed immediately after infection, in the absence of other viral functions, and one or more of the resulting gene products induces transcription of the delayed-early genes. Some delayed-early gene products, in turn, induce transcription of late genes, and finally, the very late genes are expressed under the control of previously expressed gene products from one or more of the earlier classes. One relatively well defined component of this regulatory cascade is IEI, an immediate-early gene of *Autographo californica* nuclear polyhedrosis virus (AcMNPV). IEI is pressed in the absence of other viral functions and encodes a product that stimulates the transcnption of several genes of the delayed-early class, including the 39K gene, as described in Guarino and Summers, *J. Virol.* (1986) *57*:563-571 and *J. Virol.* (1987) *61*:2091-2099 as well as late genes, as described in Guanno and Summers, *Virol*. (1988) *162*:444-451.

Immediate-early genes as described above can be used in combination with a baculovirus gene promoter region of the delayed-early category. Unlike the immediate-early genes, such delayed-early genes require the presence of other viral genes or gene products such as those of the immediate-early genes. The combination of immediate-early genes can be made with any of several delayed-early gene promoter regions such as 39K or one of the delayed-early gene promoters found on the *Hin*dIII fragment of the baculovirus genome. In the present instance, the 39 K promoter region can be linked to the foreign gene to be expressed such that expression can be further controlled by the presence of IEI, as described in L. A. Guarino and Summers (1986a), cited above; Guarino & Summers (1986b) *J. Virol*., (1986) *60*:215-223, and Guarino *et al*. (1986c), *J. Virol*. (1986) *60*:224-229.

Additionally, when a combination of immediate-early genes with a delayed-early gene promoter region is used, enhancement of the expression of heterologous genes can be realized by the presence of an enhancer sequence in direct cis linkage with the delayed-early gene promoter region. Such enhancer sequences are characterized by their enhancement of delayed-early gene expression in situations where the immediate-early gene or its product is limited. For example, the hr5 enhancer sequence can be linked directly, in cis, to the delayed-early gene promoter region, 39K, thereby enhancing the expression of the cloned heterologous DNA as described in Guarino and Summers (1986a), (1986b), and Guarino *et al*. (1986).

The polyhedrin gene is classified as a very late gene. Therefore, transcription from the polyhedrin promoter requires the previous expression of an unknown, but probably large number of other viral and cellular gene products. Because of this delayed expression of the polyhedrin promoter, state-of-the-art BEVs, such as the exemplary BEV system described by Smith and Summers in, for example, U.S. Pat. No., 4,745,051 will express foreign genes only as a result of gene expression from the rest of the viral genome, and only after the viral infection is well underway. This represents a limitation to the use of existing BEVs. The ability of the host cell to process newly synthesized proteins decreases as the baculovirus infection progresses. Thus, gene expression from the polyhedrin promoter occurs at a time when the host cell's ability to process newly synthesized proteins is potentially diminished for certain proteins such as human tissue plasminogen activator. As a consequence, the expression of secretory glycoproteins in BEV systems is complicated due to incomplete secretion of the cloned gene product, thereby trapping the cloned gene product within the cell in an incompletely processed form.

While it has been recognized that an insect signal sequence can be used to express a foreign protein that can be cleaved to produce a mature protein, the present invention is preferably practiced with a mammalian signal sequence appropriate for the gene expressed.

An exemplary insect signal sequence suitable herein is the sequence encoding for a Lepidopteran adipokinetic hormone (AKH) peptide. The AKH family consists of short blocked neuropeptides that regulate energy substrate mobilization and metabolism in insects. In an embodiment, a DNA sequence coding for a Lepidopteran *Manduca sexta* AKH signal peptide can be used. Other insect AKH signal peptides, such as those from the Orthoptera *Schistocerca gregaria* locus can also be employed to advantage. Another exemplary insect signal sequence is the sequence coding for Drosophila cuticle proteins such as CP1, CP2, CP3 or CP4.

Currently, the most commonly used transfer vector that can be used herein for introducing foreign genes into AcNPV is pAc373. Many other vectors, known to those of skill in the art, can also be used herein. Materials and methods for baculovirus/insect cell expression systems are commercially available in a kit form from companies such as Invitrogen (San Diego CA) ("MaxBac" kit). The techniques utilized herein are generally known to those skilled in the art and are fully described in Summers and Smith, A MANUAL OF METHODS FOR BACULOVIRUS VECTORS AND INSECT CELL CULTURE PROCEDURES, Texas Agricultural Experiment Station Bulletin No. 1555, Texas A&M University (1987); Smith *et al., Mol. Cell. Biol.* (1983) *3*: 2156, and Luckow and Summers (1989). These include, for example, the use of pVL985 which alters the polyhedrin start codon from ATG to ATT, and which introduces a *Bam*HI cloning site 32 basepairs downstream from the ATT, as described in Luckow and Summers, *Virology* (1989) *17*:31.

Thus, for example, for insect cell expression of the present polypeptides, the desired DNA sequence can be inserted into the transfer vector, using known techniques. An insect cell host can be cotransformed with the transfer vector containing the inserted desired DNA together with the genomic DNA of wild type baculovirus, usually by cotransfection. The vector and viral genome are allowed to recombine resulting in a recombinant virus that can be easily identified and purified. The packaged recombinant virus can be used to infect insect host cells to express a desired polypeptide.

Other methods that are applicable herein are the standard methods of insect cell culture, cotransfection and preparation of plasmids are set forth in Summers and Smith (1987), cited above. This reference also pertains to the standard methods of cloning genes into AcMNPV transfer vectors, plasmid DNA isolation, transferring genes into the AcmMNPV genome, viral DNA purification, radiolabeling recombinant proteins and preparation of insect cell culture media. The procedure for the cultivation of viruses and cells are described in Volkman and Summers, *J. Virol.* (1975) *19*:820-832 and Volkman *et al., J. Virol.* (1976) *19*:820-832.

### Expression in Mammalian Cells

Typical promoters for mammalian cell expression of the polypeptides of the invention include the SV40 early promoter, the CMV promoter, the mouse mammary tumor virus LTR promoter, the adenovirus major late promoter (Ad MLP), and the herpes simplex virus promoter, among others. Other non-viral promoters, such as a promoter derived from the murine metallothionein gene, will also find use in mammalian constructs. Mammalian expression may be either constitutive or regulated (inducible), depending on the promoter. Typically, transcription termination and polyadenylation sequences will also be present, located 3' to the translation stop codon. Preferably, a sequence for optimization of initiation of translation, located 5' to the polypeptide coding sequence, is also present. Examples of transcription terminator/polyadenylation signals include those derived from SV40, as described in Sambrook *et al.* (1989), cited previously. Introns, containing splice donor and acceptor sites, may also be designed into the constructs of the present invention.

Enhancer elements can also be used herein to increase expression levels of the mammalian constructs. Examples include the SV40 early gene enhancer, as described in Dijkema *et al.*,, *EMBO J.* (1985) *4*:761 and the enhancer/promoter derived from the long terminal repeat (LTR) of the Rous Sarcoma Virus, as described in Gorman *et al., Proc. Natl. Acad. Sci. USA* (1982b) 79:6777 and human cytomegalovirus, as described in Boshart *et al., Cell* (1985) *41*:521. A leader sequence can also be present which includes a sequence encoding a signal peptide, to provide for the secretion of the foreign protein in mammalian cells. Preferably, there are processing sites encoded between the leader fragment and the gene of interest such that the leader sequence can be cleaved either *in vivo* or *in vitro.* The adenovirus tripartite leader is an example of a leader sequence that provides for secretion of a foreign protein in mammalian cells.

Once complete, the mammalian expression vectors can be used to transform any of several mammalian cells. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei. General aspects of mammalian cell host system transformations have been described by Axel in U.S. Patent No. 4,399,216.

### Small Molecule Library Synthesis

Therapeutic agents of the invention can include organic small molecules, peptides and peptoids that have an appropriate biological activity, or that facilitate a desired biological activity in a patient. Exemplary synthesis of some small molecule libraries are described below.

Small molecule libraries are made as follows. A "library" of peptides may be synthesized and used following the methods disclosed in U.S. Patent No. 5,010,175, (the '175 patent) and in PCT WO91/17823. In method of the '175 patent, a suitable peptide synthesis support, for example, a resin, is coupled to a mixture of appropriately protected, activated amino acids.

The method described in WO91/17823 is similar. However, instead of reacting the synthesis resin with a mixture of activated amino acids, the resin is divided into twenty equal portions, or into a number of portions corresponding to the number of different amino acids to be added in that step, and each amino acid is coupled individually to its portion of resin. The resin portions are then combined, mixed, and again divided into a number of equal portions for reaction with the second amino acid. Additionally, one may maintain separate "subpools" by treating portions in parallel, rather than combining all resins at each step. This simplifies the process of determining which peptides are responsible for any observed alteration of gene expression in a responsive cell.

The methods described in WO91/17823 and U.S. Patent No. 5,194,392 enable the preparation of such pools and subpools by automated techniques in parallel, such that all synthesis and resynthesis may be performed in a matter of days.

Further alternative agents include small molecules, including peptide analogs and derivatives, that can act as stimulators or inhibitors of gene expression, or as ligands or antagonists. Some general means contemplated for the production of peptides, analogs or derivatives are outlined in CHEMISTRY AND BIOCHEMISTRY OF AMINO ACIDS, PEPTIDES, AND PROTEINS -- A SURVEY OF RECENT DEVELOPMENTS, Weinstein, B. ed., Marcell Dekker, Inc., publ. New York (1983). Moreover, substitution of D-amino acids for the normal L-stereoisomer can be carried out to increase the half-life of the molecule.

Peptoids, polymers comprised of monomer units of at least some substituted amino acids, can act as small molecule stimulators or inhibitors herein and can be synthesized as described in PCT 91/19735. Amino acid substitutes are N-alkylated derivatives of glycine, which are easily synthesized and incorporated into polypeptide chains. However, any monomer units which allow for the sequence specific synthesis of pools of diverse molecules are appropriate for use in producing peptoid molecules. The benefits of these molecules for the purpose of the invention is that they occupy different conformational space than a peptide and as such are more resistant to the action of proteases.

Peptoids are easily synthesized by standard chemical methods. A method of synthesis is the "submonomer" technique described by R. Zuckermann et al.,, J. Am. Chem. Soc. (1992) 114:10646-7. Synthesis by solid phase techniques of heterocyclic organic compounds in which N-substituted glycine monomer units forms a backbone is described in copending application entitled "Synthesis of N-Substituted Oligomers" filed on June 7, 1995 and is herein incorporated by reference in full. Combinatorial libraries of mixtures of such heterocyclic organic compounds can then be assayed for the ability to alter gene expression.

Synthesis by solid phase of other heterocyclic organic compounds in combinatorial libraries is also described in copending application U.S. Serial No. 08/485,006 entitled " Combinatorial Libraries of Substrate-Bound Cyclic Organic Compounds" filed on June 7, 1995, herein incorporated by reference in full. Highly substituted cyclic structures can be synthesized on a solid support by combining the submonomer method with powerful solution phase chemistry. Cyclic compounds containing one, two, three or more fused rings are formed by the submonomer method by first synthesizing a linear backbone followed by subsequent intramolecular or intermolecular cyclization as described in the same application.

### Ribozymes and Antisense

Where the therapeutic agent is a ribozyme, for example, a ribozyme targeting a gene encoding a target polypeptide for accomplishing a biological activity or inhibition of a certain activity in a patient, the ribozyme can be chemically synthesized or prepared in a vector for a gene therapy protocol including preparation of DNA encoding the ribozyme sequence. The synthetic ribozymes or a vector for gene therapy delivery can be encased in liposomes for delivery, or the synthetic ribozyme can be administered with a pharmaceutically acceptable carrier. A ribozyme is a polynucleotide that has the ability to catalyze the cleavage of a polynucleotide substrate. Ribozymes for inactivating a portion of HIV can be prepared and used as described in Long *et al.*,, *FASEB J. 7*: 25 (1993) and Symons, *Ann. Rev. Biochem. 61*: 641 (1992), Perrotta *et al.*,, *Biochem. 31*: 16, 17 (1992); and U.S. Pat. No. 5,225,337, U.S. Pat. No. 5,168,053, U.S. Pat. No. 5,168,053 and U.S. Pat. No. 5,116,742, Ojwang *et al.*,, *Proc. Natl. Acad. Sci. USA* 89: 10802-10806 (1992), U.S. Pat. No. 5,254,678 and in U.S. Patent No. 5,144,019, U.S. Patent No. 5,225,337, U.S. Patent No. 5,116,742, U.S. Patent No. 5,168,053. Preparation and use of such ribozyme fragments in a hammerhead structure are described by Koizumi *et al.*,, *Nucleic Acids Res.* 17:7059-7071 (1989). Preparation and use of ribozyme fragments in a hairpin structure are described by Chowrira and Burke, *Nucleic Acids Res.* 20:2835 (1992).

The hybridizing region of the ribozyme or of an antisense polynucleotide may be modified by linking the displacement arm in a linear arrangement, or alternatively, may be prepared as a branched structure as described in Horn and Urdea, *Nucleic Acids Res. 17:6959-67 (1989).* The basic structure of the ribozymes or antisense polynucleotides may also be chemically altered in ways quite familiar to those skilled in the art.

Chemically synthesized ribozymes and antisense molecules can be administered as synthetic oligonucleotide derivatives modified by monomeric units. Ribozymes and antisense molecules can also be placed in a vector and expressed intracellularly in a gene therapy protocol.

Gene therapy can be practiced according to the invention by delivery to the pericardial space genes that are under regulatory control of appropriate regulatory sequences for transformation or infection of myocytes, cells within the pericardium, cells at the epicardium, or any cells in a region of the heart accessible to an intrapericardially delivered gene. Gene therapy can be practiced as follows using coding regions for any therapeutic appropriate for treatment of a cardiovascular indication.

The polynucleotide molecule that contain a polypeptide coding sequence for use as a therapeutic agent herein, with or without the coding region for the signal sequence, can be used for treatment of a cardiovascular indication by administration thereof via gene therapy. Gene therapy strategies for delivery of such constructs can utilize viral or non-viral vector approaches in *in vivo* or *ex vivo* modality. Expression of such coding sequence can be induced using endogenous mammalian or heterologous promoters. Expression of the coding sequence *in vivo* can be either constitutive or regulated.

For delivery using viral vectors, any of a number of viral vectors conventional in the art can be used, as described in Jolly, *Cancer Gene Therapy 1:* 51-64 (1994). For example, the UPA coding sequence can be inserted into plasmids designed for expression in retroviral vectors, as described in Kimura *et al., Human Gene Therapy* (1994) *5:* 845-852, adenoviral vectors, as described in Connelly *et al., Human Gene Therapy* (1995) *6:* 185-193, adeno-associated viral vectors, as described in Kaplitt *et al., Nature Genetics* (1994) *6*: 148-153 and sindbis vectors. Recombinant retroviruses and various uses thereof have been described in numerous references including, for example, Mann et al. (*Cell 33:*153, 1983), Cane and Mulligan (*Proc. Nat'l. Acad. Sci. USA 81*:6349, 1984), Miller et al., *Human Gene Therapy* 1:5-14, 1990, U.S. Patent Nos.4,405,712; 4,861,719; 4,980,289 and PCT Application Nos. WO 89/02,468; WO 89/05,349 and WO 90/02,806, all incorporated by reference in full. Briefly, a foreign gene of interest may be incorporated into the retrovirus in place of the normal retroviral RNA. When the retrovirus injects its RNA into a cell, the foreign gene is also introduced into the cell, and may then be integrated into the host's cellular DNA as if it were the retrovirus itself. Expression of this foreign gene within the host results in expression of the foreign protein by the host cell. Briefly, numerous retroviral gene delivery vehicles may be utilized within the context of the present invention, including for example those described in EP 0,415,731; WO 90/07936; WO 94/03622; WO 93/25698; WO 93/25234; U.S. Patent No. 5,219,740; WO 9311230; WO 9310218; Vile and Hart, *Cancer Res. 53*:3860-3864, 1993; Vile and Hart, *Cancer Res. 53*:962-967, 1993; Ram et al., *Cancer Res. 53*:83-88, 1993; Takamiya et al., *J. Neurosci. Res. 33*:493-503, 1992; Baba et al., *J. Neurosurg. 79*:729-735, 1993 (U.S. Patent No. 4,777,127, GB 2,200,651, EP 0,345,242 and WO 91/02805). Recombinant retroviruses include those described in WO 91/02805.

Retroviral gene delivery vehicles of the present invention may be readily constructed from a wide variety of retroviruses, including for example, B, C, and D type retroviruses as well as spumaviruses and lentiviruses (see RNA Tumor Viruses, Second Edition, Cold Spring Harbor Laboratory, 1985). Retroviruses for the preparation or construction of retroviral gene delivery vehicles of the present invention include retroviruses selected from the group consisting of Avian Leukosis Virus, Bovine Leukemia Virus, Murine Leukemia Virus, Mink-Cell Focus-Inducing Virus, Murine Sarcoma Virus, Reticuloendotheliosis virus and Rous Sarcoma Virus. Particularly, Murine Leukemia Viruses include 4070A and 1504A (Hartley and Rowe, *J. Virol. 19*:19-25, 1976), Abelson (ATCC No. VR-999), Friend (ATCC No. VR-245), Graffi, Gross (ATCC No. VR-590), Kirsten, Harvey Sarcoma Virus and Rauscher (ATCC No. VR-998), and Moloney Murine Leukemia Virus (ATCC No. VR-190). Such retroviruses may be readily obtained from depositories or collections such as the American Type Culture Collection ("ATCC"; Rockville, Maryland), or isolated from known sources using commonly available techniques.

Any of the above retroviruses may be readily utilized in order to assemble or construct retroviral gene delivery vehicles given the disclosure provided herein, and standard recombinant techniques (*e.g*., Sambrook et al, *Molecular Cloning: A Laboratory Manual,* 2d ed., Cold Spring Harbor Laboratory Press, 1989; Kunkle, *PNAS 82*:488, 1985). In addition, within certain embodiments of the invention, portions of the retroviral gene delivery vehicles may be derived from different retroviruses. For example, retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus.

Recombinant retroviruses may be made by introducing a vector construct as discussed above, into a cell (termed a "packaging cell") which contains those elements necessary for production of infectious recombinant retrovirus which are lacking in the vector construct. A wide variety of retrovector constructs may be utilized within the present invention in order to prepare recombinant retroviruses. For example, retrovector constructs can be provided comprising a 5' LTR, a tRNA binding site, a packaging signal, one or more heterologous sequences, an origin of second strand DNA synthesis and a 3' LTR, wherein the vector construct lacks gag/pol or env coding sequences. Briefly, Long Terminal Repeats ("LTRs") are subdivided into three elements, designated U5, R and U3. These elements contain a variety of signals which are responsible for the biological activity of a retrovirus, including for example, promoter and enhancer elements which are located within U3. LTRs may be readily identified in the provirus due to their precise duplication at either end of the genome. As utilized herein, a 5' LTR should be understood to include a 5' promoter element and sufficient LTR sequence to allow reverse transcription and integration of the DNA form of the vector. The 3' LTR should be understood to include a polyadenylation signal, and sufficient LTR sequence to allow reverse transcription and integration of the DNA form of the vector. The tRNA binding site and origin of second strand DNA synthesis are also important for a retrovirus to be biologically active, and may be readily identified by one of skill in the art. For example, retroviral tRNA binds to a tRNA binding site by Watson-Crick base pairing, and is carried with the retrovirus genome into a viral particle. The tRNA is then utilized as a primer for DNA synthesis by reverse transcriptase. The tRNA binding site may be readily identified based upon its location just downstream from the 5' LTR. Similarly, the origin of second strand DNA synthesis is, as its name implies, important for the second strand DNA synthesis of a retrovirus. This region, which is also referred to as the poly-purine tract, is located just upstream of the 3' LTR. In addition to a 5' and 3' LTR, tRNA binding site, and origin of second strand DNA synthesis, certain retrovector constructs which are provided herein also comprise a packaging signal, as well as one or more nucleic acid molecules (*e.g*., heterologous sequences), each of which is discussed in more detail below.

Packaging cell lines suitable for use with the above-described retrovector constructs may be readily prepared (see U.S. Serial No. 08/240,030, filed May 9, 1994; see also WO 92/05266), and utilized to create producer cell lines (also termed vector cell lines or "VCLs") for the production of recombinant vector particles. Within embodiments of the present invention packaging cell lines are made from human (*e.g.*, HT1080 cells) or mink parent cell lines, thereby allowing production of recombinant retroviruses that are capable of surviving inactivation in human serum.

Promoters that are suitable for use with these vectors are also conventional in the art and include the Moloney retroviral LTR, CMV promoter and the mouse albumin promoter. Replication incompetent free virus can be produced and injected directly into the animal or humans or by transduction of an autologous cell *ex vivo*, followed by injection *in vivo* as described in Zatloukal *et al., Proc. Natl. Acad*. *Sci. USA* (1994) *91:* 5148-5152.

The coding sequence of, for example bFGF, VEGF, troponin C, β-adrenergic receptor, myocyte growth factor, DAF, CD59, membrane cofactor protein, ANP, dystrophin, SOD, UPA, angiogenin, TGF-α, TGF-β, G-CSF, placental growth factor, IL-8, hepatocyte growth factor, insulin-like growth factor I (IGF I), proliferin, nematode anti-coagulant protein (NAP), or any of the other recombinant therapeutics listed herein, and chimeric and analog molecules thereof, can also be inserted into plasmids for expression of the polypeptide *in vivo* or *ex vivo*. For *in vivo* therapy, the coding sequence can be delivered into the intrapericardial space by direct injection, or into pericardial tissue by delivery such as, for example, those systems described in U.S. Patent Nos. 5,137,510, 5,213,570, and 5,269,326. Promoters suitable for use in this manner include endogenous and heterologous promoters such as those described herein. Any promoter appropriate for the expression of the gene selected for the therapy is contemplated by the method of the invention. The coding sequence can be injected in a formulation comprising a buffer that can stablize the coding sequence and facilitate transduction thereof into cells and/or provide targeting, as described in Zhu *et al., Science* (1993) *261:* 209-211. Expression of such coding sequence can be induced using endogenous mammalian or heterologous promoters. Expression of the coding sequence *in vivo* can be either constitutive or regulated.

The polynucleotide encoding a desired polypeptide or ribozyme or antisense polynucleotide can also be inserted into plasmid for delivery to cells and where the polynucleotide is a coding sequence, for expression of the desired polypeptide *in vivo.* Promoters suitable for use in this manner include endogenous and heterologous promoters such as CMV. Further, a synthetic T7T7/T7 promoter can be constructed in accordance with Chen *et al.* (1994), *Nucleic Acids Res. 22:* 2114-2120, where the T7 polymerase is under the regulatory control of its own promoter and drives the transcription of polynucleotide sequence, which is also placed under the control of a T7 promoter. The polynucleotide can be injected in a formulation that can stablize the coding sequence and facilitate transduction thereof into cells and/or provide targeting, as described in Zhu *et al., Science* (1993) *261:* 209-211.

Expression of the coding sequence of a desired polypeptide or replication of a ribozyme or antisense polynucleotide *in vivo* upon delivery for gene therapy purposes by either viral or non-viral vectors can be regulated for maximal efficacy and safety by use of regulated gene expression promoters as described in Gossen *et al., Proc. Natl. Acad. Sci. USA* (1992) *89:*5547-5551. For example, the polynucleotide transcription and/or translation can be regulated by tetracycline responsive promoters. These promoters can be regulated in a positive or negative fashion by treatment with the regulator molecule.

For non-viral delivery of the coding sequence, the sequence can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, as described in Wu and Wu, *J. Biol. Chem.* (1987) *262*: 4429-4432; insulin, as described in Hucked *et al., Biochem. Pharmacol. 40:* 253-263 (1990); galactose, as described in Plank *et al., Bioconjugate Chem. 3*:533-539 (1992); lactose, as described in Midoux *et al., Nucleic Acids Res. 21:* 871-878 (1993); or transferrin, as described in Wagner *et al., Proc. Natl. Acad. Sci. USA 87*:3410-3414 (1990). Other delivery systems include the use of liposomes to encapsulate DNA comprising the gene under the control of a variety of tissue-specific or ubiquitously-active promoters, as described in Nabel *et al., Proc. Natl. Acad. Sci. USA 90:* 11307-11311 (1993), and Philip *et al., Mol. Cell Biol. 14:* 2411-2418 (1994). Further non-viral delivery suitable for use includes mechanical delivery systems such as the biolistic approach, as described in Woffendin *et al., Proc. Natl. Acad*. *Sci. USA* (1994) *91(24):* 11581-11585. Moreover, the coding sequence and the product of expression of such can be delivered through deposition of photopolymerized hydrogel materials. Other conventional methods for gene delivery that can be used for delivery of the coding sequence include, for example, use of hand held gene transfer particle gun, as described in U.S. 5,149,655; use of ionizing radiation for activating transferred gene, as described in U.S. 5,206,152 and PCT application WO 92/11033. The aforementioned are not to the exclusion of additional means of facilitating of nucleic acid uptake that rely on nucleic charge neutralization or fusion with cell membranes or facilitate uptake, for example.

Administration a gene for expression in the patient for a non-immunological effect, or a non-coding polynucleotide sequence, can be accomplished by use of a polypeptide, a peptide, a conjugate, a liposome, a lipid, a viral vector, for example, a retroviral vector a non-viral vector.

Polycationic molecules, lipids, liposomes, polyanionic molecules, or polymer conjugates conjugated to the polynucleotide can facilitate non-viral delivery of DNA or RNA. For example, polycationic agents for gene delivery include: polylysine, polyarginine, polyornithine, and protamine. Other examples include histones, protamines, human serum albumin, DNA binding proteins, non-histone chromosomal proteins, coat proteins from DNA viruses, such as φX174, transcriptional factors also contain domains that bind DNA and therefore may be useful as nucleic aid condensing agents, for example, C/CEBP, c-*jun*, c-*fos*, AP-1, AP-2, AP-3, CPF, Prot-1, Sp-1, Oct-1, Oct-2, CREP, and TFIID contain basic domains that bind DNA sequences. Organic polycationic agents include: spermine, spermidine, and purtrescine. The dimensions and of the physical properties of a polycationic agent can be extrapolated from the list above, to construct other polypeptide polycationic agents or to produce synthetic polycationic agents.

Typically, the polycationic agents exhibit a predicted isoelectric point of at least 9, excluding the terminal groups. The agents contain, excluding the terminal groups, at least 20% basically or positively charged monomers; more typically, at least 25%; more typically, 30%; even more typically, at least 33%; even more typically at least 40%; even more typically, at least 50%; even more typically, at least 60%. Additionally, the agents do not comprises greater than 5% acidic monomers and preferably none. The charge density and composition of the polycationic agent can be altered to accommodate the specific nucleic acid sequence, type, and other components included with the complex of nucleic acids and polycationic agent.

A group of neutral polymers are of the general formula of compounds of the instant invention as follows: 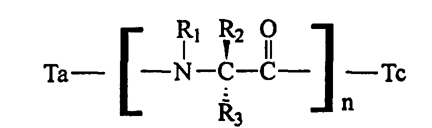 A preferred subset of these compounds comprise where R₂ is hydrogen. Even more preferred are polymers comprising at least one natural amino acid. Also preferred are polymers where R₂ and R₃ are hydrogen, also referred to as poly N-substituted glycines or poly NSGs.

Monomers will be the general formula as the polycationic monomers with the following structure:

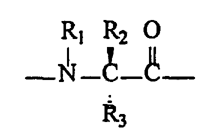

Generally, R₁, R₂, and R₃ are organic moieties each with a molecular weight from 1 to 250 daltons. More typically, the molecular weight is no more than 200; even more typically, no more than 175. Typically, the each monomer comprises one hydrogen at R₁, R₂, or R₃. More, typically, either R1 and R3 are both hydrogen, the structure of a L-amino acid; or R2 and R3 are both hydrogen, the structure of a NSG. Monomers to be utilized in the neutral agents can be either positively or negatively charged. Also, neutral substituents can also be utilized. The polymers exhibit no net positive or negative charge, excluding the terminal groups.

Degradation sites can be incorporated into the polymers by using naturally occurring amino acid substituents in monomers when R₁ and R₃ are hydrogen. Naturally occurring amino acids and analogues are designated D-amino acids to indicate the chirality of these molecules. L-amino acids can also incorporated as monomers into the neutral polymers. The substituents of L-amino acids can be, for example, the same as those named for the D-amino acids. Also, preferred are NSG to be incorporated as monomers. Preferred monomers are those that capable of forming hydrogen bonds with the polynucleotides to be delivered.

Polymers can be linked together incorporating terminating groups or side chains that permit cross-linking of the polymers. For example, polymers can be linked by a disulfide bond. Other terminating groups useful for coupling polymers include, carbonate, urea, and the like. Additional components can be included in the polycationic agents of the instant invention, such as targeting ligands. Such additional groups can facilitate endocytosis of the desired nucleic acids or aid binding of the nucleic acids to the cell surface.

Polypeptides can be incorporated into the polycationic agents. Examples include, without limitation: asioloorosomucoid (ASOR); transferrin; asialoglycoproteins; antibodies; antibody fragments; ferritin; interleukins; interferons, granulocyte macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms are useful, such as the 17 amino acid peptide from the circumsporozoite protein of plasmodium falciparum known as RII.

In addition, lipoproteins can be incorporated into the polycationic agent, such as low density lipoprotein, high density lipoprotein, or very low density lipoprotein. Mutants, fragments, or fusions of these proteins can also be used. Other groups that can be incorporated include without limitation: hormones, steroids, androgens, estrogens, thyroid hormone, or vitamins, folic acid. Folic acid can be incorporated into the polycationic agent according, for example, to Mislick, *et al., T.J. Bioconjugate Chem. 6:* 512 (1995). Also, the polycationic agents of the instant invention can be chemically conjugated with polyalkylene glycol. In a preferred embodiment, the polyalkylene glycol is polyethlylene glycol. PEG can be incorporated with a polycation agent according, for example, to Lu, et al., Int. J. Pept. Protein Res. 43: 127 (1994).

In addition, the polycationic agent can be chemically conjugated with mono-, di-, or polysaccharide. In a preferred embodiment of this aspect, the polysaccharide is dextran. These additional groups can be incorporated within the polycationic agent. For example, R₁, R₂, and R₃ can be a substituent that is capable of being activated to cross link with any one of the above groups. For example, a thiol group could be included to cross link with another group to form a disulfide bond.

The terminal groups of the instant polycationic agents can be chosen as convenient. For example, to enhance the targeting properties of the polycationic agent, any of the additional groups described above can be incorporated as terminal groups.

The additional groups described above can be incorporated at the terminus of the polycationic agent. For example, the polycationic agent can be (1) acylated with a variety of carboxylic acids; (2) sulfonylated with sulfonyl chlorides; or (3) derivatized with isocyanates or isothiocyanates. Once activated, the terminus can be reacted with any of the above-mentioned groups, such as a polypeptide, such as low density lipoprotein, or folic acid.

One means of adding a terminal group to the polycationic agent is, for example, is
(1) to acylate the amino terminus with Fmoc-amino-hexanoic acid; and
(2) to remove the protecting group, Fmoc, to generate a primary amine, which can be further functionalized. Alternatively, the amino-terminal groups can include, without limitation: acyl, such as acetyl, benzoyl; or sulfonyl, such as dansyl. Carboxy terminal groups can include, for example, amide or alkyl amide.

The following is a solid phase method for the synthesis of NSGs, which can be generally used for a wide variety of side-chain substituents. This method can be performed utilizing automated peptide synthesis instrumentation to permit rapid synthesis of polycationic agents of interest. Such instruments are commercially available from, for example, Applied Biosystems and Milligen.

A method of synthesis is to assemble the monomer from two submonomers in the course of extending a polymer comprising a NSG monomer. This technique is described in Zuckermann *et al*., J Amer Chem Soc 114(26): 10646-10647 (1992) and Zuckermann *et al*., PCT WO94/06451. The NSGs can also be considered to be an alternating condensation of copolymer of an acylating agent and an amine.

The direction of polymer synthesis with the submonomers occurs in the carboxy to amino direction. The solid-phase assembly for each monomer, in the course of polymer formation, eliminates the need for Nα-protected monomers, as only reactive side-chain functionalities need to be protected. Each monomer addition comprises two steps, an acylation step and a nucleophilic displacement step: (1) acylation of a secondary amine bound to the support with an acylating agent comprising a leaving group capable of nucleophilic displacement by an amine and a carbonyl group, preferably carboxyl. An example is a haloacetic acid; and (2) nucleophilic displacement of the leaving group with a sufficient amount of a submonomer comprising a primary amino group to introduce a side-chain. The amino group containing submonomer can be an alkoxyamine, semicarbazide, acyl hydrazide, substituted hydrazine or the like.

Acylation can be activated with carbodimide or other suitable carboxylate activation method. The efficiency of the displacement is modulated by the choice of halide, e.g., I>C1. Protection of aliphatic hydroxyl groups, carboxylic acids, carboxy, thiol, amino, some heterocyles, and other reactive side-chain functionalities is preferred to minimize undesired side reactions. However, the mild reactivity of some side-chain moieties toward displacement or acylation may allow their use without protection., e.g., indole, imidazole, and phenol.

NSGs can also be constructed utilizing a three step method for assembling each monomer as the polymer is extended. The backbone of the monomer if first extended by acylation step followed by a nucleophilic displacement. The side chain is introduced by a second acylation step. The backbone of the monomer is assembled in the first two steps of the synthesis cycle. The first reaction is an acylation step where the carbonyl group of the acylating agent reacts with an amine. The acylating agent comprises a carbonyl group; a backbone, Rₐ; and a leaving group, L. Preferably, the carbonyl group is carboxyl.

The second step is a nucleophilic displacement of the leaving group by the first amino group of the displacing agent. The displacing agent comprises a first and a second amino group and a backbone, R_{d}. The first amino group is a primary amine, and the second step produces a secondary amine.

The third step is another acylation in which the another acylating submonomer reacts with the first amino group of the displacing agent to produce a tertiary amide. The acylation agent comprises of a carbonyl group; an optional linker; and a sidechain. Preferably, the carbonyl group is carboxyl.

The polycationic agent/polynucleotide complexes, whether or not encapsulated in liposomes, may be administered in pharmaceutical compositions. The pharmaceutical compositions will comprise therapeutically effective amount of nucleic acids. An effective dose for DNA delivery is from about 0.01 mg/ kg to 50 mg/kg or 0.05 mg/kg to about 10 mg/kg of the DNA constructs in the individual to which it is administered.

Additional agents can be included with the desired polynucleotides to be delivered, for delivery in either in a gene therapy protocol, or in a nucleic acid vaccination protocol. These additional agents can facilitate, for example, endocytosis of the desired nucleic acids or aid binding of the nucleic acids to the cell surface.

Polypeptides can facilitate DNA delivery and include, for example: asioloorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

Certain hormones such as for example, steroids, androgens, estrogens, thyroid hormone, or the vitamin, folic acid, can aid in nucleic acid delivery.

Also, polyalkylene glycol can be included with the desired polynucleotides, such as, for example, the polyalkylene glycol is polyethlylene glycol. In addition, mono-, di-, or polysaccharides can be included, for example, the polysaccharide dextran or DEAE-dextran, and poly(lactide-co-glycolide)

The desired polynucleotide can also be encapsulated in lipids or packaged in liposomes prior to delivery to the patient. Lipid encapsulation is generally accomplished using liposomes which are able to stably bind or entrap and retain nucleic acid. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight, *Biochim. Biophys. Acta.* (1991) *1097*:1-17; Straubinger *et al*., in METHODS OF ENZYMOLOGY (1983), Vol. 101, pp. 512-527.

Liposomal preparations for use in the instant invention include cationic (positively charged), anionic (negatively charged) and neutral preparations. Cationic liposomes have been shown to mediate intracellular delivery of plasmid DNA (Felgner *et al., Proc. Natl. Acad. Sci. USA* (1987) *84*:7413-7416); mRNA (Malone *et al., Proc. Natl. Acad. Sci. USA* (1989) *86*:6077-6081); and purified transcription factors (Debs *et al., J. Biol. Chem.* (1990) *265*:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner *et al., Proc. Natl. Acad. Sci. USA* (1987) *84*:7413-7416). Other commercially available liposomes include transfectace (DDAB/DOPE) and DOTAP/DOPE (Boerhinger). Other cationic liposomes can be prepared from readily available materials using techniques well known in the art. See, e.g., Szoka *et al., Proc. Natl. Acad. Sci. USA* (1978) *75*:4194-4198; PCT Publication No. WO 90/11092 for a description of the synthesis of DOTAP (1,2-bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See, e.g., Straubinger *et al*., in METHODS OF ENZYMOLOGY (1983), Vol. 101, pp. 512-527; Szoka *et al., Proc. Natl. Acad. Sci. USA* (1978) *75*:4194-4198; Papahadjopoulos *et al., Biochim. Biophys. Acta* (1975) *394*:483; Wilson *et al., Cell* (1979) *17*:77); Deamer and Bangham, *Biochim. Biophys. Acta* (1976) *443*:629; Ostro *et al., Biochem. Biophys. Res. Commun*. (1977) *76*:836; Fraley *et al., Proc. Natl. Acad. Sci. USA* (1979) *76*:3348); Enoch and Strittmatter, *Proc. Natl. Acad. Sci. USA* (1979) *76*:145); Fraley *et al., J. Biol. Chem.* (1980) *255*:10431; Szoka and Papahadjopoulos, *Proc. Natl. Acad. Sci. USA* (1978) *75*:145; and Schaefer-Ridder *et al., Science* (1982) *215*:166.

In addition, lipoproteins can be included with the polynucleotide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are included with the polynucleotide to be delivered, no other targeting ligand is included in the composition.

If lipoproteins are included with the desired polynucleotides to be delivered, preferably, the composition comprises: lipoprotein, a polynucleotide, and a polynucleotide binding molecule.

Naturally occurring lipoproteins are made up of a lipid and a protein portion. The protein portions of such molecules are known as apoproteins. At the present, apoproteins A, B, C, D, and E have been isolated and identified. At least two of these contain several proteins, designated by Roman numerals, Al, All, AIV; CI, CII, CIII. A lipoprotein can comprise more than one apoprotein. For example, naturally occurring chylomicrons comprises of A, B, C, and E, over time these lipoproteins lose A and acquire C and E apoproteins. VLDL comprises A, B, C, and E apoproteins, LDL comprises apoprotein B; and HDL comprises apoproteins A, C, and E. The amino acid of these apoproteins are known and are described in, for example, Breslow, *Ann Rev. Biochem 54*: 699 (1985); Law et al., *Adv. Exp Med. Biol. 151*: 162 (1986); Chen *et al., J Biol Chem 261*: 12918 (1986); Kane *et al., Proc Natl Acad Sci USA 77*: 2465 (1980); and Utermann *et al. Hum Genet 65: 232:* (1984).

Lipoproteins contain a variety of lipids including, triglycerides, cholesterol (free and esters), and phospholipids. The composition of the lipids varies in naturally occurring lipoproteins. For example, chylomicrons comprise mainly triglycerides. A more detailed description of the lipid content of naturally occurring lipoproteins can be found, for example, in Meth. Enzym. 128 (1986). The composition of the lipids are chosen to aid in conformation of the apoprotein for receptor binding activity. The composition of lipids can also be chosen to facilitate hydrophobic interaction and association with the polynucleotide binding molecule.

Naturally occurring lipoproteins can be isolated from serum by ultracentrifugation, for instance. Such methods are described in Meth. Enzy., supra; Pitas et al., J. Biochem. 255: 5454-5460 (1980); and Mahey et al., J Clin. Invest 64: 743-750 (1979). Lipoproteins can also be produced by in vitro or recombinant methods by expression of the apoprotein genes in a desired host cell. See, for example, Atkinson et al., Annu Rev Biophys Chem 15: 403 (1986) and Radding et al., Biochim Biophys Acta 30: 443 (1958). Lipoproteins can also be purchased from commercial suppliers, such as Biomedical Technologies, Inc., Stoughton, Massachusetts, USA.

Mutants, fragments and fusion of the naturally occurring apoproteins are useful for delivery of polynucleotides. These polypeptides will retain more than about 80% amino acid identity; more typically, more than about 85%; even more typically, at least 90%. Preferably, these polypeptides will exhibit more than about 92% amino acid sequence identity with naturally occurring lipoproteins or fragment thereof; more preferably, more than about 94%; even more preferably, more than about 96%; even more preferably, more than about 98%; even more preferably, more than about 99% sequence identity.

Such mutants, fragments and fusions can be constructed by altering the polynucleotides encoding the desired lipoproteins by recombinant DNA techniques. See, for example, Sambrook et al., (1989) Molecular Cloning, A Laboratory Manual, 2d edition (Cold Spring Harbor Press, Cold Spring Harbor, New York). These polynucleotides can be inserted into expression vectors and host cells can be utilized to produce the desired apoprotein.

In addition, naturally occurring lipoproteins, mutants, fragments, and fusions can be chemically altered. For example, acetylated LDL has biological activity. See, for example, Nagelkerke *et al., J. Biol. Chem. 258(20):* 12221-12227 (1983); Weisgraber *et al., J. Biol. Chem. 253*: 9053-9062 (1978); Voyta *et al., J. Cell Biol. 99*: 2034-2040 (1984); Goldstein *et al., Proc. Natl. Acad. Sci. USA 76*: 333-337 (1979); and Pitas, *Arterosclerosis 1:* 177-185 (1981). Chemically modified lipoproteins can also be purchased from commercial suppliers, such as Biomedical Technologies, Inc., Stoughton, Massachusetts, USA.

All of these polypeptides exhibit receptor binding properties of naturally occurring lipoproteins. Usually, such polypeptides exhibit at least about 20% receptor binding of naturally occurring lipoproteins. More typically, the polypeptides exhibit at least about 40%, even more typically the polypeptides exhibit at least about 60%; even more typically, at least about 70%; even more typically, at least about 80%; even more typically, at least about 85%; even more typically, at least about 90%; even more typically, at least about 95% receptor binding of the naturally occurring lipoproteins.

Typically, lipoproteins are in an effective amount to increase the frequency of incorporation of polynucleotides into a cell. Such an amount increases the frequency of incorporation of polynucleotides into a cell is at least 10% greater than the frequency of incorporation of naked polynucleotides; more usually, at least 15% greater; even more usually, 20% greater; even more usually, at least 30%. The increase can be between 40 to 100%, and even 1000% and 10000% increase.

A polynucleotide binding molecule refers to those compounds that associate with polynucleotides, and the association is not sequence specific. For example, such molecules can (1) aid in neutralizing the electrical charge of polynucleotide, or (2) facilitate condensation of nucleotides, or (3) inhibit serum or nuclease degradation. Optionally, polynucleotide binding molecules can interact with lipoproteins by either hydrophobic association or by charge. Polynucleotide binding molecules include, without limitation, polypeptides, mineral compounds, vitamins, etc.

Examples of polynucleotide binding molecules include: polylysine, polyarginine, polyornithine, and protamine. Examples of organic polycations include: spermine, spermidine, and purtrescine. Other examples include histones, protamines, human serum albumin, DNA binding proteins, non-histone chromosomal proteins, coat proteins from DNA viruses, such as φX174, transcriptional factors also contain domains that bind DNA and therefore may be useful as nucleic aid condensing agents. Briefly, transcriptional factors such as C/CEBP, *c-jun*, *c-fos*, AP-1, AP-2, AP-3, CPF, Prot-1, Sp-1, Oct-1, Oct-2, CREP, and TFIID contain basic domains that bind DNA sequences.

Examples of other positively charged moieties include polybrene, DEAE-dextran, and cationic lipids. Useful cationic lipids and liposomes are described above. Lipids and liposomes are not used in this aspect of the invention to encapsulate both polynucleotide and lipoprotein. The lipoprotein must be exposed to bind the its cell surface receptor.

Other synthetic compounds that are capable of binding negatively charged polynucleotides are useful, such as polymers of N-substituted glycines.

In a composition with a lipoprotein, the polynucleotide binding molecule can be in an amount effective to neutralize the polynucleotide. However, the polynucleotide binding molecule also can be in excess of an effective amount to neutralize the polynucleotide to be delivered. Such an excess can produce a net positive electrical charge when complexed with the polynucleotides to be delivered. The positively charged complex can then interact lipoproteins that comprise negatively charged lipids, such as phospholipids.

Typically, the polynucleotide binding molecule is in excess when the amount is 10% greater than the amount to neutralize the polynucleotide charge; more typically, the amount is 50% greater; even more typically, 100% greater; even more typically, 150% greater; even more typically, 200% greater; even more typically, 500% greater; even more typically, 20,000% greater; even more typically, 22,000% greater; even more typically, 25,000% greater; even more typically, 30,000% greater; even more typically, more than 40,000% greater than the amount effective to neutralize the electrical charge of the desired polynucleotide.

To practice the invention, the diagnosis of a cardiovascular condition is made, and the appropriate therapeutic agent or agents and dosages are determined on the basis of the diagnosis. The invention is practiced to prevent, reduce or treat a coronary condition. Diagnosis can include diagnosis of the coronary condition and can be made using standard techniques.

A therapeutic agent can be administered to a patient with a coronary condition, or a patient at risk for developing a cardiovascular condition, in a protocol that includes administration of several therapeutic agents. Any of these therapeutic agents can be incorporated into an appropriate pharmaceutical composition that includes a pharmaceutically acceptable carrier for the agent. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in REMINGTON'S PHARMACEUTICAL SCIENCES (Mack Pub. Co., N.J. 1991). Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier. The term "liposomes" refers to, for example, the liposome compositions described in U.S. Patent No. 5,422,120, WO 95/13796, WO 94/23697, WO 91/14445 and EP 524,968 B1. Liposomes may be pharmaceutical carriers for the small molecules, polypeptides or polynucleotides of the invention, or for combination of these therapeutics.

Additionally, administration of the therapeutic agents used in the invention can be accomplished for example, in a simaltaneous administration, in sequential administration, and with the same or different pharmaceutically acceptable carriers, as is appropriate for best accomplishing the goal of improving the patient's condition. Further, a therapeutic composition can be administered that includes all the therapeutic agents necessary to achieve the therapeutic goals of the therapy. Co-administration of more than one therapeutic agent can be accomplished by administration of at least one first therapeutic agent, and by administration of at least one second therapeutic agent. The combined administration of the first, and second therapeutic agents make-up a co-administration. The timing of the co-administration can be simultaneous or sequential. Where the two therapeutic agents are in the same pharmaceutical composition, the administration of the the composition can be considered a co-admininstration.

Administration of a therapeutic agent used in the invention, includes administering a therapeutically effective dose of the therapeutic agent, by a means considered or empirically deduced to be effective for inducing the desired, therapeutic effect in the patient. Both the dose and the administration means can be determined based on the specific qualities of the therapeutic, the condition of the patient, the progression of the disease, and other relevant factors. Administration of the therapeutic agents used in the invention can include, for example, any administration targeted to releasing the therapeutic agent in the pericardial space, including for example parenteral administration, including injection, catheterization, laser-created perfusion channels, a particle gun, and a pump. Parenteral administration can be, for example, intravenous, subcutaneous, intradermal, or intramuscular, administration.

Methodology for the physical delivery of a therapeutic agent to the pericardial space includes intrapericardial internal and external entry. Internal entry is characterized by entry through the atrium or ventricle of the heart. This can mean an entry into the right or the left portion of the heart. External entry includes, for example, open chest surgery, minimal invasion surgery (MIS) and percutaneous administration. The percutaneous administration can be accomplished by a device appropriate for such procedure, including but not limited to needles, catheters, cannulas, and trocars.

The therapeutic agents used in the invention can be administered in a therapeutically effective dosage and amount, in the process of a therapeutically effective protocol for treatment of the patient. The initial and any subsequent dosages administered will depend upon the patient's age, weight, condition, and the disease, disorder or biological condition being treated. Depending on the therapeutic, the dosage and protocol for administration will vary, and the dosage will also depend on the method of administration selected, for example, local or systemic administration.

For polypeptide therapeutics, for example, the dosage can be in the range of about 5µg to about 50µg/kg of patient body weight, also about 50 µg to about 5 mg/kg, also about 100 µg to about 500 µg/kg of patient body weight, and about 200 to about 250 ug/kg.

For polynucleotide therapeutics, depending on the expression of the polynucleotide in the patient, for tissue targeted administration, vectors containing expressable constructs of coding sequences, or non-coding sequences can be administered in a range of about 100 ng to about 200 mg of DNA for local administration in a gene therapy protocol, also about 500 ng to about 50 mg, also about 1 ug to about 2 mg of DNA, about 5 ug of DNA to about 500 ug of DNA, and about 20 ug to about 100 ug during a local administration in a gene therapy protocol, and for example, a dosage of about 500 ug, per injection or administration.

Non-coding sequences that act by a catalytic mechanism, for example, catalytically active ribozymes may require lower doses than non-coding sequences that are held to the restrictions of stoichometry, as in the case of, for example, antisense molecules, although expression limitations of the ribozymes may again raise the dosage requirements of ribozymes being expressed *in vivo* in order that they achieve efficacy in the patient. Factors such as method of action and efficacy of transformation and expression are therefore considerations that will effect the dosage required for ultimate efficacy for DNA and nucleic acids. Where greater expression is desired, over a larger area of tissue, larger amounts of DNA or the same amounts readministered in a successive protocol of administrations, or several administrations to different adjacent or close tissue portions of for example, a tumor site, may be required to effect a positive therapeutic outcome.

For administration of small molecule therapeutics, depending on the potency of the small molecule, the dosage may vary. For a very potent inhibitor, microgram (µ) amounts per kilogram of patient may be sufficient, for example, in the range of about 1 µg/kg to about 500 mg/kg of patient weight, and about 100 µg/kg to about 5 mg/kg, and about 1 µg/kg to about 50 µg/kg, and, for example, about 10 ug/kg. For administration of peptides and peptoids the potency also affects the dosage, and may be in the range of about 1µg/kg to about 500 mg/kg of patient weight, and about 100 µg/kg to about 5 mg/kg, and about 1 µg/kg to about 50 µg/kg, and a usual dose might be about 10 ug/kg.

In all cases, routine experimentation in clinical trials will determine specific ranges for optimal therapeutic effect, for each therapeutic, each administrative protocol, and administration to specific patients will also be adjusted to within effective and safe ranges depending on the patient condition and responsiveness to initial administrations.

The invention applies to any cardiovascular indication, for example a diagnosis of: (1) atherosclerosis, and conditions that predispose one to pathological atherosclerotic plaque development in the coronary arteries including lipid/cholesterol deposition, macrophage/inflammatory cell recruitment, plaque rupture, thrombosis, platelet deposition, neointimal proliferation; (2) ischemic syndromes and attendant syndromes, including but not limited to myocardial infarction or ischemia, stable and unstable angina, coronary artery restenosis following percutaneous transluminal, coronary angioplasty, reperfusion injury; (3) cardiomyopathies, including but not limited to cardiomyopathies caused by ischemic syndromes, cardiotoxins such as alcohol and chemotherapeutic agents like adriamycin, infections, such as viral, cytomegalovirus (CMV), and parasitic (trypanosoma cruzi), hypertension, metabolic diseases, (including but not limited to uremia, beriberi, glycogen storage disease), radiation, neuromuscular disease (such as Duchenne's muscular dystrophy), infiltrative diseases (including but not limited to sarcoidosis, hemochromatosis, amyloidosis, Fabry's disease, Hurler's syndrome), trauma, and idiopathic causes; (4) a/dysrrhythmias (including but not limited to a/dysrrhythmias resulting from the same causes listed above for cardiomyopathies); (5) infections (including bacterial, viral, fungal, and parasitic causes); (6) cardiac tumors; (7) inflammatory conditions (including but not limited to myocarditis, pericarditis, endocarditis, immune cardiac rejection and conditions resulting from idiopathic, autoimmune, or connective tissue diseases); and (8) hypertension.

The therapeutic agent selected for practice of the invention can be a drug or a polynucleotide, or a combination of the two, or a combination of more than one drug, or a combination of more than one polynucleotide. Where a drug is selected, the appropriate formulation of the drug for minimizing a detrimental immunological response and for maximizing the effectiveness of the drug, including perfusion of the drug, is also selected. Some appropriate formulations include buffers, excipients, gels, matrices and polymers known in the art of drug delivery. Appropriate formulations for drugs used in the practice of the invention also include liposomal preparations such as, for example, those disclosed in U.S. Patent No. 5,422,120, WO 95/13796, WO 94/23697, WO 91/14445 and EP 0 524 968-B1, particularly including heterovesicular liposomal preparations. Formulations of liposomes provide an increased and sustained delivery of drugs much improved from the prior formulations, and such formulations are particularly well-suited to the method of this invention.

Where the therapeutic agent selected for treatment is a polynucleotide that is then administered to the pericardial space and expressed in the heart tissue, including but not limited to, for example, pericardial tissue, myocardial tissue, epicardial tissue, or perivascular tissue, the polynucleotide is isolated and placed in a vector. The vector is, for example, a viral vector, or a plasmid vector. The regulatory sequences are any regulatory sequence appropriate the polynucleotide and other parameters comprising the gene therapy, and can be any appropriate regulatory sequence or combination of sequences. The polynucleotides may be presented into the pericardial space in any formulation commonly known in the art including buffers, excipients, gels, matrices and polymers. Appropriate formulations for the polynucleotides administered intrapericardially in the practice of the invention also include liposomal preparations such as, for example, those disclosed in U.S. Patent No. 5,422,120, WO 95/13796, WO 94/23697, WO 91/14445 and EP 524,968 B1, particularly including the heterovesicular liposomal preparations disclosed in these patents and applications.

The polynucleotide for delivery into the pericardial space can be prepared by any method conventional in the art, such as that described in Barr *et al., Gene Therapy* (1994) *1*:51-58, using replication deficient adenoviral vectors. The polynucleotide for intrapericardial delivery may be linked to tissue specific promoters or leader sequences for expression in cardiac muscle cells, for example, the untranslated leader sequence of dystrophin DNA, or regulatory regions muscle creative kinase gene such as that described in Cox *et al., Nature* (1993) *364*:725-729.

Where the therapeutic agent used in the invention is a combination of a drug and a polynucleotide, appropriate formulations containing pharmaceutically acceptable carriers are also prepared for the combination. In addition, the drug and the polynucleotide can be prepared in separate formulations, of the same formulation, and can be administered simultaneously or consecutively.

The drugs and polynucleotides that are appropriate for use in the invention include any cardiac drug or any polynucleotide that is known to or is expected to prevent, reduce or treat a cardiovascular condition. Some examples of such drugs and polynucleotides are listed below. They include, but are not limited to:
(1) an agent used for treatment of coronary artery occlusion or reocclusion (as occurs in atherosclerosis, thrombosis, or restenosis), including an inhibitor of lipid/cholesterol synthesis/deposition (such as, for example, fish oil, HMG), and an inhibitor of macrophage/inflammatory cell recruitment or activation, such as, for example, NF-κB inhibitors like IκB, pyrolidine dithiocarbamate, and N-acetyl cysteine, microtubule inhibitors like colchicine and Taxol, and any antiinflammatory agent, any antithrombotic agent, any antiplatelet agent, and an inhibitor of neointimal proliferation;
(2) an agent directed at the prevention, treatment, or reduction of attendant effects of the myocardial ischemic syndromes, including, for example,
   (a) an anti-apoptotic agent, such as, for example, an inhibitor of interleukin 1b converting enzyme;
   (b) a thrombolytic agent, such as, for example, tissue plasminogen activator (TPA), urokinase plasminogen activator (UPA), urokinase, streptokinase, an inhibitor of α2 plasmin inhibitor, nematode anti-coagulant protein (NAP), nematode anti-coagulant protein (NAP), and an inhibitor of plasminogen activator inhibitor-1,
   (c) a pro-angiogenic agent, such as, for example, basic and acidic fibroblast growth factor, FGF-5, vascular endothelial growth factor, angiogenin, transforming growth factor alpha and beta, tumor necrosis factor alpha, platelet derived growth factor, placental growth factor, hepatocyte growth factor, and proliferin;
   (d) a complement blocker, such as, for example, decay accelerating factor;
   (e) an inhibitor of reperfusion injury, such as, for example, CAB-2;
   (f) a calcium channel blocker, such as, for example, diltiazem;
   (g) a beta-blocker, such as, for example, propranolol;
   (h) an afterload reducer, such as, for example, hydralazine;
   (i) a preload reducer, such as, for example, nitroglycerin;
   (j) a vasoactive agent such as, for example, nitric oxide (NO), a nitric oxide inhibitor, or an inhibitor of NO synthase:
   (k) an anti-thrombotic agent, such as, for example, tissue factor pathway inhibitor, heparin, hirudin, protein C, protein S, anti-thrombin III, tick anti-coagulant peptide (TAP), and antistasin;
   (l) an antiplatelet agent, such as, for example, a glycoprotein IIb/IIIa antagonist;
   (m) a cyclooxygenase inhibitor, such as, for example, aspirin or non-steroidal anti-inflammatory agents, prostacylin, or agents that increase platelet cAMP;
   (n) an anti-proliferative agent, such as, for example, ribozymes, antisense oligonucleotides, antibodies, protein, peptide, or small molecule inhibitors against c-myb, ras/raf, PI3 kinase, cyclins, or such as, for example, suicide proteins/genes such as, for example, herpes thymidine kinase or proapoptotic proteins/genes like fas, faf, interleukin 1β converting enzyme;
   (o) an inhibitor of reactive oxygen metabolites, such as, for example, superoxide dismutase, N-acetyl cysteine, pyrolidine dithiocarbamate, vitamin E derivatives, and metal ion chelators; and
   (p) an antiangiogenic agent, such as, for example, platelet factor 4, thrombospondin, a tissue inhibitor of a metalloproteinase, prolactin, bFGF soluble receptor, angiostatin, TFG-β, interferon-α, and proliferin-related protein;
(3) an agent to prevent, reduce, or treat cardiomyopathy, including
   (a) the above list for ischemic syndromes; and also including
   (b) a contractility improving agent, such as, for example, digitalis;
   (c) a myocyte growth factor, such as insulin-like growth factor 1 (IGF-1);
   (d) a cardioprotective agent, such as, for example, Cardioxane;
   (e) an iron-chelating agent, such as, for example, desferoxamine;
   (f) an anti-viral or anti-parasitic agent,
   (g) a free radical scavenger, such as, for example, superoxide dismutase; and
   (h) the replacement of genes or proteins which may be deficient or downregulated during the development of cardiomyopathy, such as, for example, troponin C or the beta-adrenergic receptor;
(4) an antiarrhythmic agent including, for example, adenosine, quinidine, propranolol, digoxin, lidocaine, bretylium, amiodarone, and verapamil;
(5) an antibiotic agent, including, for example, antibacterial, antivirals anti-fungal, and antiparasitic agents;
(6) an anti-tumor agent, including, for example, chemotherapeutic agents or radiation sensitizers or radioactive implants;
(7) an anti-inflammatory agent including, for example, an anti-inflammatory or immunomodulating agent, such as, for example, non-steroidal anti-inflammatory agents, cyclosporin, chemotherapeutic agents, and complement inhibitors; and
(8) an anti-hypertensive agent, including but not limited to, for example, hydralazine, propranolol, atrial naturetic peptide, and endothelin antagonists.

Administration of the therapeutic agent into the pericardial space is accomplished by a means appropriate to the cardiovascular condition of the patient, the therapeutic agent and its formulation, and the goals of the treatment. Repeated or continuous administration over a period of time is also contemplated by the invention. In general, any method appropriate for local delivery of a therapeutic to any part of the body is appropriate for administration into the pericardial space. These methods include, for example, injection, catheterization, laser-created perfusion channels, cannulization, a particle gun, and a pump.

In order to maximize the perfusion of the myocardial tissues during the treatment by the method of the invention, the invention can be practiced by first administering to the pericardial space proangiogenic factors to increase vascularization of myocardial tissue, formulating agents such that tissue penetration by the agent is enhanced, or by first creating perfusion channels in the myocardial tissue with a laser. Subsequently, the therapeutic agent is administered into the pericardial space, and the perfusion to tissue and cells of the agent is increased.

Also, a therapeutically effective amount of an anti-inflammatory agent to counteract an inflammatory response to administration of the polynucleotide can be administered either before, contemporaneously with, or after administration of the polynucleotide.

The therapeutic agent used in the invention can be a polypeptide, polynucleotide or other drug that is an anti-apoptotic agent, a thrombolytic agent, a pro-angiogenic agent, an anti-arrythmic agent, a contractility improving agent, a complement blocker, an inhibitor of reperfusion injury, an calcium channel blocker, a beta-blocker, an afterload reducer, a preload reducer, a vasoactive agent, an anti-thrombotic agent, an anti-platelet agent, anti-proliferative agent, an anti-inflammatory agent, an immunomodulating agent, an immunosuppressive agent, an inhibitor of reactive oxygen metabolites, an anti-angiogenic agent, a vasoactive agent, a cardioprotective agent, an iron-chelating agent, an anti-hypertensive agent, an anti-integrin agent, a pro-apoptotic agent, an anti-viral agent, an anti-parasitic agent, a free radical scavenger, a protein that may be deficient or downregulated during the development of cardiomyopathy, a biologically active fragment thereof, or a chimera thereof.

Efficacy of myocardial revascularization by laser can be augmented by administering a therapeutic agent, such as a proangiogenic agent like basis fibroblast growth factor (bFGF), or a gene encoding bFGF, into the pericardial space in close proximity to the laser revascularization procedure.

Treatment of cardiomyopathy can be accomplished by administering to the pericardial space agents that improve contractility of the myocardial tissue. For example, it is known that severe arterial hypotension that is unresponsive to volume replacement can be treated in titrated doses with continous infusion of dopamine at an amount and concentration of about 400 mg/250ml of 5% D/W (1.6mg/ml) beginning at 3 to 5 ug/kg/min, and including also epinephrine, norepinephrine or phenylephrine as described in THE MERCK MANUAL, (Berkow Ed., Merck Res. Lab., Rahway, N.J. 16th Edition (1992), pg. 534. In addition, other adminstrative protocols are possible, for example, administering a polynucleotide encoding L-aromatic amino acid decarboxylase to the pericardial space. L-aromatic amino acid decarboxylase converts L-Dopa to dopamine which improves contractility of myocardial tissue. L-aromatic amino acid decarboxylase can be administered either in the polynucleotide form for expression in cells in the pericardial space, the polynucleotide form transfected into cells removed from the pericardial space which are then readministered to the pericardial space for expression of the L-aromatic amino acid decarboxylase, or in the polypeptide form of L-aromatic amino acid decarboxylase. L-aromatic amino acid decarboxylase allows the metabolism of L-Dopa to dopamine, an active agent in improving cardiac muscle contractility. Coincident with or in combination with an administration of L-aromatic amino acid decarboxylase, can thus be administered any combination of the following: L-Dopa systemically or L-Dopa locally to the pericardial space. Additionally, for example, the elements which make up L-Dopa can be administered: including tyrosine and the enzyme that converts tyrosine to L-Dopa called tyrosine hydroxylase. Tyrosine hydroxylase can be administered as a protein having activity or as a polynucleotide for expression in the cells of the pericardial space, or some cells of the pericardium can be removed and transfected with the tyrosine hydroxylase and the transfected cells can be returned to the pericardial space for expression of the enzyme therein.

Any polynucleotide for administration to the patient can be expressed *in vitro* (in cells removed from the patient and replaced after transformation into the pericardial space) or *in vivo* (in the patient by administration of the polynucleotide into the pericardial space) from a viral vector, including, for example, a vector derived from a retrovirus, an adenovirus, an adeno-associated virus, a herpes virus, an alpha virus, a semliki forest virus, or a sindbis virus. The viral vector can include a gene, for example a suicide gene, for the purpose of inactivating expression of the polynucleotide at an appropriate or necessary time. Thus the viral vector capable of expressing the polynucleotide therapeutic can also contain, for example, a thymidine kinase gene from the Herpes simplex virus. Gancyclovir can be administered to the patient and a cell expressing the thymidine kinase will enable phosphorylation of gancyclovir which makes gancyclovir become toxic which kills the cell. Thus, the expression of the polynucleotide of interest is stopped. Other genes which when combined with a pro-drug like gancyclovir can also be used for this purpose, for example the PNP gene.

A chaperone molecule can be administered before, contemporaneously with or after administration of the polynucleotide therapeutic, and the chaperone molecule can be, for example, a heat shock protein, such as, for example hsp70. Further, the polynucleotide being expressed in the cardiac patient can be linked to an inducible promoter, for example a heart tissue specific promoter, for the purpose of, for example, for ensuring expression of the polynucleotide only in the myocyotes adjacent to the pericardial space. Additionally, for the purpose of effectively delivering the polynucleotide to heart tissue, the polynucleotide can be flanked by nucleotide sequences suitable for integration into genome of myocytes.

Further objects, features, and advantages of the present invention will become apparent from the detailed description. It should be understood, however, that the detailed description, while indicating preferred embodiments of the invention, is given by way of illustration only, since various changes and modifications within the spirit and scope of the invention will become apparent to those skilled in the art from this detailed description. Also, the invention is not limited by any theories of mechanism of the invention.

### Example 1

### Administration of a Growth Factor to the Pericardial Space

A bFGF polypeptide is placed in a pharmaceutical composition in a lipsomal formulation containing also heparin sulfate. The pharmaceutical composition is delivered by laparoscopic cannulation to the pericardial space of a cardiac patient by external delivery through the chest cavity to a patient who has suffered a mycardial infarction. The patient is monitored for improved heart function and readministration of the bFGF polypeptidee with heparin sulfate polypeptide is repeated for several administrations as necessary for patient recovery.

### Example 2

### Lysing Pericardial/Epicardial Adhesions

A patient is diagnosed with a coronary condition manifesting adhesions between the pericardium and epicardial surface of the heart due to ischemia. To solve the problem of incomplete access to all regions of the pericadial space, a deposit of TPA polypeptide which is capable of lysing pericardial/epicardial adhesions is made. The TPA is prepared for delivery in a gel pharmaceutical composition. The regions of ischemia are located by imaging and the delivery is accomplished by external entry from outside the chest into the chest cavity in the region of the ischemic lesions. Afterwards the reaction with TPA occurs, the pericardial space is filled with sterile saline for a short period of time to further release the adhesions. The patient is then ready for an administration of a second therapeutic agent, which is a pro-angiogenic factor combination therapeutic agent that includes VEGF and bFGF in the same pharmaceutical composition. The agents are administered by catheter via an external entry through the chest wall.

### Example 3

### Local Delivery of a Growth Factor to the Pericardial Space

Regions of the heart exhibiting ischemic myocardium at risk for infarction resulting in myocardial injury are identified by a thallium scan. bFGF and VEGF polypeptides are prepared in a pharmaceutical composition that includes a gel and a ligand for an integrin present on the cell surfaces of myocytes exhiting cell injury. The agent is administered through the ventricle wall or the atrium wall as is appropriate for the location of the ischemic myocardium.

## Claims

1. Use of a first therapeutic agent in the manufacture of a pharmaceutical composition for treatment or prevention of a coronary condition wherein the pharmaceutical composition is administered to the pericardial space of a patient simultaneously with, or sequentially after enhancing access of the first therapeutic agent to myocardial tissue.

2. Use of a first therapeutic agent in the manufacture of a pharmaceutical composition for treatment or prevention of a coronary condition wherein the pharmaceutical composition is administered to the pericardial space of a patient and the pharmaceutical composition comprises an agent which enhances access to myocardial tissue.

3. Use according to claim 1 or 2 wherein the pharmaceutical composition further comprises a second therapeutic agent.

4. Use according to claim 1, 2 or 3 wherein the first and/or second therapeutic agent is selected from a polypeptide, a polynucleotide, a small organic molecule, a peptide, and a peptoid.

5. Use according to claim 1, 2 or 3 wherein the first and/or second therapeutic agent comprises a fibroblast growth factor polypeptide (FGF) or insulin-like growth factor-I polypeptide (IGF-1).

6. Use according to claim 5 wherein the FGF polypeptide is selected from bFGF, aFGF and FGF-5.

7. Use according to claim 1, 2 or 3, wherein the first and/or second therapeutic agent is selected from an anti-apoptotic agent, a thrombolytic agent, a pro-angiogenic agent, an anti-arrythmic agent, a contractility improving agent, a complement blocker, an inhibitor of reperfusion injury, a calcium channel blocker, a beta-blocker, an afterload reducer, a preload reducer, a vasoactive agent, an anti-thrombotic agent, an anti-platelet agent, an anti-proliferative agent, an antiflammatory agent, an immunomodulating agent, an immunosuppressive agent, an inhibitor of reactive oxygen metabolites, an anti-angiogenic agent, a myocyte growth factor, a vasoactive agent, a cardioprotective agent, an iron-chelating agent, an anti-hypertensive agent, an anti-integrin agent, a pro-apoptotic agent, an anti-viral agent, an anti-parasitic agent, a free radical scavenger, an anti-tumor agent, a protein that may be deficient or downregulated during development of cardiomyopathy, and biologically active derivatives thereof.

8. Use according to claim 7 wherein the protein that may be deficient or downregulated during development of cardiomyopathy is troponin C.

9. Use according to claim 7 wherein the first and/or second therapeutic agent is an anti-tumor agent and the anti-tumor agent is selected from a chemotherapeutic agent, a radiation sensitizer, and a radioactive implant.

10. Use according to claim 1, 2 or 3, wherein the first and/or second therapeutic agent is an inhibitor.

11. Use according to claim 10, wherein the inhibitor is selected from an inhibitor of lipid or cholesterol syntheses or deposition, an inhibitor of macrophage or inflammatory cell recruitment or activation, a microtubule inhibitor, an anti-inflammatory agent, an anti-thrombotic agent, an anti-platelet agent, and an inhibitor of neointimal proliferation.

12. Use according to claim 10 wherein the inhibitor is an inhibitor of NFκB.

13. Use according to claim 12 wherein the inhibitor of NFκB is selected from IKB, PDTC, NAC metal chelators and anti-oxidants.

14. Use according to claim 10 wherein the inhibitor is selected from a protein, a peptide, an oligopeptide or a small molecule.

15. Use according to claim 7 wherein the first and/or second therapeutic agent is an anti-proliferative agent and the anti-proliferative agent is selected from a ribozyme, an antisense oligonucleotide, an antibody, an inhibitor against one of c-myb, ras, raf, P13 kinase, and cyclin, a suicide protein, a suicide gene, a proapoptolic protein, and a proapoptotic gene.

16. Use according to claim 7 wherein the first and/or second therapeutic agent is a proapoptotic protein and the propoptotic protein is selected form fas, fab and interieuldn 1b converting enzyme.

17. Use according to claim 7 wherein the first and/or second therapeutic agent is an anti-angiogenic agent and the antiangiogenic agent is selected from factor-4, thrombospondin, a tissue inhibitor of a metaloproteinase, angiostatin, TFG-β, interferon-α, a proliferin-related protein, a biologically active fragment thereof, and a chimera thereof.

18. Use according to claim 7 wherein the first and/or second therapeutic agent is an antibiotic and the antibiotic is selected from an anti-viral agent, anti-trypanosomal agent, anti-bacterial agent, and an anti-fungal agent.

19. Use according to claim 7 wherein the first and/or second therapeutic agent is an immunomodulating agent and the immunomodulating agent is selected from a cytotoxic agent, a steroid, cyclosporin and a complement inhibitor.

20. Use according to claim 19 wherein the immunomodulating agent is a complement inhibitor and the complement inhibitor is selected from DAF, CAB2, a biologically active fragment thereof, and a chimera thereof.

21. Use according to claim 7 wherein the first and/or second therapeutic agent is an antiflammatory agent and the anuflammatory agent is selected from a steroid, a non-steroidal antiinflammatory agent, cyclosporin, a chemotherapeutic agent, and a complement inhibitor.

22. Use according to claim 7 wherein the first and/or second therapeutic agent is an anti-arrhythmic agent and the anti-arrythmic agent is selected from adenosine, quinidine, propranolol, digoxin, lidocaine, bretylium, amiodarone, and verapamil.

23. Use according to claim 7 wherein the first and/or second therapeutic agent is an anti-hyertensive agent, and the antihypertensive agent is selected from hydralazine, propanol, atrial naturetic peptide, and an endothelin antagonist.

24. Use according to claim 1, 2 or 3 wherein the first and/or second therapeutic agent is a polypeptide selected from tissue plasminogen activator (tPA), an inhibitor of interleukin 1β converting enzyme, urokinase plasminogen activator (uPA), urokinase, streptokinase, an inhibitor of α-2 plasmin inhibitor, an inhibitor of plasminogen activator inhibitor-1 (PAI-1), basic fibroblast growth factor (VEGF), angiogenin, transforming growth factor α (TGF-α), transforming growth factor β (TGF-β), tumor necrosis factor-α (TNF-α), platelet derived growth factor (PDGF), placental growth factor (PGF), hepatocyte growth factor, proliferin, decay accelerating factor, CAB-2, tissue factor pathway inhibitor (TFPI), heparin, hirudin, protein C, protein S, anti-thrombin III, tick anti-coagulant peptide (TAP), anti-stasin, glycoprotein IIb/IIa antagonist, antibodies, Herpes thymidine kinase, fas, faf, platelet factor 4, thrombospondin, a tissue inhibitor of a metalloproteinase, prolactin, bFGF soluble receptor, a proliferin-related protein, myocyte growth factor, superoxide dismutase (SOD), troponin C, beta-adrenergic receptor, insulin-like growth factor I (IGF-1), nematode anti-coagulant protein (NAP), biologically active fragments thereof, and chimeras thereof.

25. Use according to any one of claims 1 to 24 wherein administration to the pericardial space is accomplished by internal entry or external entry.

26. Use according to claim 25 wherein the method of internal entry is selected from entry through the left atrium, entry through the right ventricle, and entry through the left ventricle.

27. Use according to claim 25 wherein the method of external entry is selected from an open chest procedure, minimally invasive surgery (MIS), and percuntaneous entry.

28. Use according to claim 27 wherein the percentaneous entry is facilitated by a device selected from a needle, catheter, cannula and trocar.

29. Use according to any one of claims 1 to 24 wherein administration to the pericardial space is accomplished by a procedure selected from injection, catheterization, creation of laser-created perfusion channels, cannulization, use of a particle gun, and use of a pump.

30. Use according to any one of the preceding claims wherein the coronary condition is a condition selected from coronary artery occlusion, ischemic syndromes, cardiomyopathy, arrhythmia, dysrrhythmia, infection, and an inflammatory condition.

31. Use according to claim 30, wherein the coronary condition is coronary artery occlusion and the coronary occlusion results from or is associated with lipid/cholesterol deposition, macrophage/inflammatory cell recruitment, plaque rupture, thrombosis, platelet deposition, or neointimal proliferation.

32. Use according to claim 30 wherein the coronary condition is an ischemic syndrome and the ischemic syndrome results from or is associated with myocardial infarction, stable angina, unstable angina, coronary artery restenosis or reperfusion injury.

33. Use according to claim 30 whereon the coronary condition is cardiomyopathy and the cardiomyopathy results from or associated with a isochemic syndrome, a cardiotoxin, an infection, hypertension, a metabolic disease, radiation, a neuromuscular disease, trauma, or an idiopathic cause.

34. Use according to claim 33 wherein the cardiomyopathy results from an infiltrative disease and the infiltrative disease is selected from sarcoidosis, hemochromatosis, amyloidosis, Fabry's disease, and Hurler's syndrome.

35. Use according to claim 33 wherein the cardiomyopathy results from a metabolic disease and the metabolic disease is selected from uremia, beriberi, and glycogen storage disease.

36. Use according to claim 30 wherein the coronary condition is an arrythmia or a dysrythmia resulting from or associated with an ischemic syndrome, a cardiotoxin adromycin, an infection, hypertension, a metabolic disease,. radiation, a neuromuscular disease, an infiltrative disease, trauma, or an idiopathic cause.

37. Use according to claim 30 wherein the coronary condition is an infection caused by a pathogenic agent selected from a bacterium, a virus, a fungus, and a parasite.

38. Use according to claim 30 wherein the coronary condition is an inflammatory condition and the inflammatory condition is associated with myocarditis, pericarditis, endocarditis, immune cardiac rejection, and an inflammatory conditions resulting from one of idiopathic, autoimmune, or a connective tissue diease.

39. Use according to any one of the preceding claims wherein enhancing access comprises a step selected from increasing penetration of myocardial tissue by the first and/or second therapeutic agent, and creating perfusion channels.

40. Use according to claim 39 wherein increasing penetration comprises an administration of a proangiogenic factor to a pericardial space of a patient to increase vascularization of myocardial tissue.

41. Use according to claim 39 wherein increasing penetration comprises administering the first and/or second therapeutic agent in a formulation that increases tissue penetration of the first and/or second therapeutic agent.

42. Use according to any one of the preceding claims wherein the pharmaceutical composition comprises a component selected from a liposome, cyclodextrin liposome, heterovesicular liposome, a synthetic membrane vesicle, a gel, a polymer, an excipient, matrices, a charged particle and a buffer.

43. Use according to claim 42 wherein the component is a gel and the gel comprises Focalgel.

44. Use according to claim 42 wherein the component is a liposome and the liposome comprises Depofoam®.

45. Use of a therapeutic agent in the manufacture of a pharmaceutical composition for use in a method of treating cardiac muscle tissue which method comprises:
a. identifying an infarct or ischemic zone,
b. accessing a pericardial space in the region of the infarct or ischemic zone; and
c. delivering the pharmaceutical composition to the region of the infarct or ischemic zone,
wherein access of the therapeutic agent to the region of the infarct or ischemic zone is enhanced by creating perfusion channels, administering a proangiogenic factor to the pericardial space or including in the pharmaceutical composition an agent that increases tissue penetration of the therapeutic agent.

46. Use according to claim 45 wherein the pharmaceutical composition further comprises a component selected from an adherent gel, a polymer, a targeting ligand, a targeting antibody, and an agent active at low pH.

47. Use according to claim 45 or 46 wherein the pharmaceutical composition comprises a combination of at least two therapeutic agents.

48. Use according to claim 45, 46 or 47 wherein the therapeutic agent is as defined in any one of claims 3 to 24.

49. Use of an agent capable of lysing a pericardial/epicardial adhesion for the manufacture of a medicament for use in a method of accessing the pericardial space prior to administration of a therapeutic agent to the pericardial space, wherein the method of accessing the pericardial space further comprises the step of expanding the pericardial space.

50. Use according to claim 49 wherein expanding the pericardial space comprises temporary administration of liquid or gas.

51. Use according to claim 49 or 50 wherein the agent capable of lysing a pericardial/epicardial adhesion is an agent selected from any fibrinolytic agent, tissue plasminogen activator (tPA), streptokinase, urokinase, collagenase, and a matrix metalloprotease.

52. Use according to claim 49 wherein the therapeutic agent is as defined in any one of claims 3 to 24.

53. Use according to claim 1 wherein the first therapeutic agent comprises L-amino acid decarboxylase.

54. Use according to claim 53 wherein the pharmaceutical composition further comprises a second therapeutic agent selected from L-Dopa, tyrosine, a polynucleotide encoding tyrosine hydroxylase, and a tyrosine hydroxylase polypeptide.

55. Use according to claim 1 wherein the first therapeutic agent comprises vascular epithelial growth factor (VEGF), a biologically active fragment thereof or a chimera thereof.

## Patentansprüche

1. Verwendung eines ersten therapeutischen Mittels zur Herstellung einer pharmazeutischen Zusammensetzung für die Behandlung oder Prävention eines koronaren Krankheitsbildes, wobei die pharmazeutische Zusammensetzung in den perikardialen Raum eines Patienten gleichzeitig mit oder nach einer Verstärkung des Zugangs des ersten therapeutischen Mittels zum Myokardgewebe verabreicht wird.

2. Verwendung eines ersten therapeutischen Mittels zur Herstellung einer pharmazeutischen Zusammensetzung für die Behandlung oder Prävention eines koronaren Krankheitsbildes, wobei die pharmazeutische Zusammensetzung in den perikardialen Raum eines Patienten verabreicht wird und die pharmazeutische Zusammensetzung ein Mittel umfasst, dass den Zugang zum Myokardgewebe verstärkt.

3. Verwendung nach Anspruch 1, wobei die pharmazeutische Zusammensetzung weiter ein zweites therapeutisches Mittel umfasst.

4. Verwendung nach Anspruch 1, 2 oder 3, wobei das erste und/oder das zweite therapeutische Mittel aus einer Gruppe ausgewählt wird, bestehend aus einem Polypeptid, einem Polynukleotid, einem kleinen organischen Molekül, einem Peptid und einem Peptoid.

5. Verwendung nach Anspruch 1, 2 oder 3, wobei das erste und/oder zweite therapeutische Mittel ein Polypeptid des Fibroblasben-Wachstumsfaktors (FGF) oder des Insulin-ähnlichen Wachstumsfaktor-I (IGF-1) umfassen/umfasst.

6. Verwendung nach Anspruch 5, wobei das FGF-Polypeptid aus einer Gruppe ausgewählt wird, bestehend aus bFGF, aFGF und FGF-5.

7. Verwendung nach Anspruch 1, 2 oder 3, wobei das erste und/oder zweite therapeutische Mittel aus einer Gruppe ausgewählt wird, bestehend aus einem antiapoptotischen Mittel, einem thrombolytischen Mittel, einem proangiogenetischen Mittel, einem antiarrhythmischen Mittel, einem Kontraktions-verbessernden Mittel, einem Komplement-Blocker, einem Inhibitor der Reperfusionsverletzung, einem Calciumkanal-Bocker, einem Betablocker, einem Reduktionsmittel für die Nachbelastung (Afterload), einem Reduktionsmittel für die Vorbelastung (Preload), einem vasoaktiven Mittel, einem antithrombotischen Mittel, einem Anti-Thrombocytenmittel, einem antiproliferativen Mittel, einem entzündungshemmenden Mittel, einem immunmodulierenden Mittel, einem immunsuppressiven Mittel, einem Inhibitor von reaktiven Sauerstoff-Metaboliten, einem antiangiogenetischen Mittel, einem Myocyten-Wachstumsfaktor, einem vasoaktiven Mittel, einem kardioprotektiven Mittel, einem Eisenchelat-bildenden Mittel, einem antihypertensiven Mittel, einem Anti-Integrin-Mittel, einem proapoptotischen Mittel, einem antiviralen Mittel, einem antiparasitären Mittel, einem Mittel zum Einfangen freier Radikale, einem Anti-Tumor-Mittel, einem Protein, das während der Entwicklung von Kardiomyopathie defizient oder herabreguliert sein kann, und biologisch aktiven Derivaten davon.

8. Verwendung nach Anspruch 7, wobei das Protein, das während der Entwicklung von Kardiomyopathie defizient oder herabreguliert sein kann, Troponin-C ist.

9. Verwendung nach Anspruch 7, wobei das erste und/oder zweite therapeutische Mittel ein Anti-Tumor-Mittel sind/ist und das Anti-Tumor-Mittel aus einer Gruppe ausgewählt wird, bestehend aus einem chemotherapeutischen Mittel, einem Strahlen-Sensibilisierungsmittel und einem radioaktiven Implantat.

10. Verwendung nach Anspruch 1, 2 oder 3, wobei das erste und/oder zweite therapeutische Mittel ein Inhibitor sind/ist.

11. Verwendung nach Anspruch 10, wobei der Inhibitor aus einer Gruppe ausgewählt wird, bestehend aus einem Inhibitor der Lipid- oder Cholesterinsynthesen oder -ablagerung, einem Inhibitor der Makropbagen- oder Entzündungszellenrekrutierung oder -aktivierung, einem Inhibitor des Mikrotubulus, einem entzündungshemmenden Mittel, einem antithrombotischen Mittel, einem Anti-Thrombocytenmittel und einem Inhibitor der Neointima-Proliferation.

12. Verwendung nach Anspruch 10, wobei der Inhibitor ein NFκB-Inhibitor ist.

13. Verwendung nach Anspruch 12, wobei der NFκB-Inhibitor aus einer Gruppe ausgewählt wird, bestehend aus IκB, PDTC, NAC, Metallchelatbildner und Antioxidanzien.

14. Verwendung nach Anspruch 10, wobei der Inhibitor aus einer Gruppe ausgewählt wird, bestehend aus einem Protein, einem Peptid, einem Oligopeptid oder einem kleinen Molekül.

15. Verwendung nach Anspruch 7, wobei das erste und/oder zweite therapeutische Mittel ein antiproliferatives Mittel sind/ist und das antiproliferative Mittel aus einer Gruppe ausgewählt wird, bestehend aus einem Ribozym, einem Anti-Sense-Oligonukleotid, einem Antikörper, einem Inhibitor gegen ein c-myb, ras, raf, P13-Kinase und Cyklin, einem Suizidprotein, einem Suizidgen, einem proapoptotischen Protein und einem proapoptotischen Gen.

16. Verwendung nach Anspruch 7, wobei das erste und/oder zweite therapeutische Mittel ein proapoptotisches Protein sind/ist und das proapoptotische Protein aus einer Gruppe ausgewählt wird, bestehend aus fas, fab und Interleukinlb-umwandelndem-Enzym.

17. Verwendung nach Anspruch 7, wobei das erste und/oder zweite therapeutische Mittel ein antiangiogenetisches Mittel sind/ist und das emtiangiogenetische Mittel aus einer Gruppe ausgewählt wird, bestehend aus Faktor-4, Thrombospondin, einem Gewebeinhibitor einer Metallproteinase, Angiostatin, TFG-β, Interferon-α, einem Proliferin-verwandten Protein, einem biologisch aktiven Fragment davon und einem Chimären davon.

18. Verwendung nach Anspruch 7, wobei das erste und/oder zweite therapeutische Mittel ein Antibiotikum sind/ist und das Antibiotikum aus einer Gruppe ausgewählt wird, bestehend aus einem antiviralen Mittel, einem antitrypanosomalen Mittel, einem antibakteriellen Mittel und einem antifungalen Mittel.

19. Verwendung nach Anspruch 7, wobei das erste und/oder zweite therapeutische Mittel ein immunmodulierendes Mittel sind/ist und das immunmodulierende Mittel aus einer Gruppe ausgewählt wird, bestehend aus einem cytotoxischen Mittel, einem Steroid, Cyclosporin und einem Komplement-Inhibitor.

20. Verwendung nach Anspruch 19, wobei das immunmodulierende Mittel ein Komplement-Inhibitor ist und der Komplement-Inhibitor aus einer Gruppe ausgewählt wird, bestehend aus DAF, CAB2, einem biologisch aktiven Fragment davon und einem Chimären davon.

21. Verwendung nach Anspruch 7, wobei das erste und/oder zweite therapeutische Mittel ein entzündungshemmendes Mittel sind/ist und das entzündungshemmende Mittel aus einer Gruppe ausgewählt wird, bestehend aus einem Steroid, einem nichtsteroiden entzündungshemmenden Mittel, Cyclosporin. einem chemotherapeutischen Mittel und einem Komplement-Inhibitor.

22. Verwendung nach Anspruch 7, wobei das erste und/oder zweite therapeutische Mittel ein antiarrhythmisches Mittel sind/ist und das antiarrhythmische Mittel aus einer Gruppe ausgewählt wird, bestehend aus Adenosin, Chinidin, Propranolol, Digoxin, Lidocain, Bretylium, Amiodaron und Verapamil.

23. Verwendung nach Anspruch 7, wobei das erste und/oder zweite therapeutische Mittel ein antihypertensives Mittel sind/ist und das antihypertensive Mittel aus einer Gruppe ausgewählt wird, bestehend aus Hydralazin, Propanol, atrialem natriuretischen Peptid und einem Endothelin-Antagonisten.

24. Verwendung nach Anspruch 1, 2 oder 3, wobei das erste und/oder zweite therapeutische Mittel ein Polypeptid sind/ist, ausgewählt aus einer Gruppe, bestehend aus Gewebe-Plasminogen-Aktivator (tPA), einem Inhibitor des Interleukin-1β-umwandelnden-Enzyms, Urokinase-Plasminogen-Aktivator (uPA), Urokinase, Streptokinase, einem Inhibitor des α-2-Plasmin-Inhibitors, einem Inhibitor des PlasminogenAktivator-Inhibitor-1 (PAI-1), basischem Fibroblasten-Wachstumsfaktor (VEGF), Angiogenin, transformierenden Wachstumsfaktor-α (TGF-α), transformierenden Wachstumsfaktor-β (TGF-β), Tumor-Nekrose-Faktor-α (TNF-α), Plättchenwachstumsfaktor (PDGF), Placenta-Wachstumsfaktor (PGF), Hepatocyten-Wachstumsfaktor, Proliferin, decay accelerating factor, CAB-2, Gewebefaktorbahn-Inhibitor (TFPI), Heparin, Hirudin, Protein-C, Protein-S, Anti-Thrombin-III, Zecken-Antikoagulans-Peptid (TAP), Anti-Stasin, Glycoprotein IIb/IIa-Antagonist, Antikörper, Herpes-Thymidin-Kinase, fas, faf, Plättchenfaktor-4, Thrombospondin, einem Gewebeinhibitor einer Metallproteinase, Prolactin, löslichem bFGF-Rezeptor, einem Proliferin-verwandten Protein, Myocyten-Wachstumsfaktor, Superoxid-Dismutase (SOD), Troponin-C, beta-adrenergen Rezeptor, Insulin-ähnlichen Wachstumsfaktor-I (IGF-1), Nematoden-Antikoagulans-Protein (NAP), biologisch aktiven Fragmenten davon und Chimären davon.

25. Verwendung nach einem der Ansprüche 1 bis 24, wobei die Verabreichung in den perikardialen Raum durch internes Einführen oder externes Einführen vollzogen wird.

26. Verwendung nach Anspruch 25, wobei das Verfahren der internen Einführung aus einer Gruppe ausgewählt wird, bestehend aus einer Einführung durch das linke Atrium, einer Einführung durch den rechten Ventrikel und einer Einführung durch den linken Ventrikel.

27. Verwendung nach Anspruch 25, wobei das Verfahren der externen Einführung aus einer Gruppe ausgewählt wird, bestehend aus einer Technik bei offenem Brustkorb, einer minimal invasiven Chirurgie (MIS) und einer perkutaner Einführung.

28. Verwendung nach Anspruch 27, wobei die perkutane Einführung mit einer Vorrichtung, ausgewählt aus einer Gruppe, bestehend aus einer Nadel, einem Katheter, einer Kanüle und einem Trokar, erleichtert wird.

29. Verwendung nach einem der Ansprüche 1 bis 24, wobei die Verabreichung in den perikardialen Raum durch ein Verfahren, ausgewählt aus einer Gruppe, bestehend aus Injektion, Katheterisierung, Bildung von mit einem Laser gebildeten Perfusionskanälen, Kanülisierung, Verwendung einer Partikelpistole und Verwendung einer Pumpe, durchgeführt wird.

30. Verwendung nach einem der vorhergehenden Ansprüchen, wobei das koronare Krankheitsbild aus einer Gruppe ausgewählt wird, bestehend aus Koronararterienverschluss, I-schämiesyndromen, Kardiomyopathie, Arrhythmie, Dysrhythmie, Infektion und einem Entzündungszustand.

31. Verwendung nach Anspruch 30, wobei das koronare Krankheitsbild ein Koronararterienverschluss ist und der Koronararterienverschluss auf Lipid-/Cholesterin-Ablagerung, eine Makrophagen-/Entzündungszellenrekrutierung, P1aquebruch, Thrombose, Plättchenablagerung oder Neointima-Proliferation zurückzuführen ist oder damit verbunden ist.

32. Verwendung nach Anspruch 30, wobei das koronare Krankheitsbild ein Ischämiesyndrom ist und das Ischämiesyndrom auf einen myokardialen Infarkt, eine chronische Angina, eine nicht-chronische Angina, eine Koronararterienrestenose oder eine Reperfusionsverletzung zurückzuführen ist oder damit verbunden ist.

33. Verwendung nach Anspruch 30, wobei das koronare Krankheitsbild eine Kardiomyopathie ist und die Kardiomyopathie auf ein Ischämiesyndrom, ein Kardiotoxin, eine Infektion, eine Hypertension, eine metabolitische Erkrankung, Strahlung, eine neuromuskuläre Erkrankung, Trauma oder eine idiopathische Ursache zurückzuführen ist oder damit verbunden ist.

34. verwendung nach Anspruch 33, wobei die Kardiomayopathie auf eine infiltrative Erkrankung zurückzuführen ist und die infiltrative Erkrankung aus einer Gruppe ausgewählt wird, bestehend aus Sarkoidose, Hämochromatose, Amyloidose, Fabrysche Erkrankung und Hurler-Syndrom.

35. Verwendung nach Anspruch 33, wobei die Kardiomyopathie auf eine metabolische Erkrankung zurückzuführen ist und die metabolische Erkrankung aus einer Gruppe ausgewählt wird, bestehend aus Urämie, Beriberi und Glykogenspeicher-Erkrankung.

36. Verwendung nach Anspruch 30, wobei das koronare Krankheitsbild eine Arrhythmie oder eine Dysrhythmie ist, die auf ein Ischämie-Syndrom, Kardiotoxin, Adromycin, eine Infektion, Hypertension, eine metabolische Erkrankung, Strahlung, eine neuromuskuläre Erkrankung, eine infiltrative Erkrankung, Trauma zurückzuführen ist oder damit verbunden ist oder eine idiopathische Ursache hat.

37. Verwendung nach Anspruch 30, wobei das koronare Krankheitsbild eine Infektion ist, die durch ein pathogenes Mittel, ausgewählt aus einer Gruppe, bestehend aus einem Bakterium, einem Virus, einem Pilz und einem Parasiten, verursacht wurde.

38. Verwendung nach Anspruch 30, wobei das koronare Krankheitsbild ein Entzündungszustand ist und der Entzündungszustand mit einer Myokarditis, Perikarditis, Endokarditis, immune Herzabstoßung und einem Entzündungszustand, der auf eine idiopathische, Autoimmun- oder Bindegewebserkrankung zurückzuführen ist, verbunden ist.

39. Verwendung nach einem der vorhergehenden Ansprüchen,
wobei der verstärkte Zugang einen Schritt, ausgewählt aus einer Gruppe, bestehend aus erhöhter Penetration des Myokardialgewebes durch das erste und/oder zweite therapeutische Mittel und Bildung von Perfusionskanälen, umfasst.

40. Verwendung nach Anspruch 39, wobei die erhöhte Penetration eine Verabreichung eines proangiogenetischen Faktors in einen perikardialen Raum eines Patienten umfasst, um die Vaskularisierung des Myokardgewebes zu erhöhen.

41. Verfahren nach Anspruch 39, wobei die erhöhte Penetration die Verabreichung eines ersten und/oder zweiten therapeutischen Mittels in einer Zubereitung, welche die Gewebepenetration des ersten und/oder zweiten therapeutischen Mittels erhöht, umfasst.

42. Verfahren nach einem der vorhergehenden Ansprüchen, wobei die pharmazeutische Zusammensetzung einen Bestandteil, ausgewählt aus einer Gruppe bestehend aus einem Liposom, Cyclodextrin-Liposom, heterovesikulärem Liposom, einem synthetischen Membranvesikel, einem Gel, einem Polymer, einem Arzneimittelträger, Matrices, einem geladenen Teilchen und einem Puffer, umfasst.

43. Verwendung nach Anspruch 42, wobei der Bestandteil ein Gel ist und das Gel Focalgel umfasst.

44. Verwendung nach Anspruch 42, wobei der Bestandteil ein Liposom ist und das Liposom Depofoam® umfasst.

45. Verwendung eines therapeutischen Mittels für die Herstellung einer pharmazeutischen Zusammensetzung zur Verwendung in einem Verfahren für die Behandlung von Herzmuskelgewebe, wobei das Verfahren umfasst:
a. Identifizierung eines Infarkts oder einer Ischämiezone,
b. Zugänglichmachen eines perikardialen Raums in der Infarktregion oder der Ischämiezone; und
c. Abgabe der pharmazeutischen Zusammensetzung in die Infarktregion oder in die Ischämiezone,
wobei der Zugang des therapeutischen Mittels in die Ischämiezone durch Bildung von Perfusionskanälen, Verabreichung eines proangiogenetischen Faktors in den perkardialen Raum oder durch Einschluß eines Mittels, das die Gewebepenetration des therapeutischen Mittels erhöht, in die pharmazeutischen Zusammensetzung, erhöht wird.

46. Verwendung nach Anspruch 45, wobei die pharmazeutische Zusammensetzung weiter einen Bestandteil, ausgewählt aus einer Gruppe, bestehend aus einem haftenden Gel, einem Polymer, einem Ziel liganden, einem Zielantikörper und einem Mittel, das bei niedrigem pH aktiv ist, umfasst.

47. Verwendung nach Anspruch 45 oder 46, wobei die pharmazeutische Zusammensetzung eine Kombination von mindestens zwei therapeutischen Mittel umfasst.

48. Verwendung nach Anspruch 45, 46 oder 47, wobei das therapeutische Mittel in einem der Ansprüche 3 bis 24 definiert wird.

49. Verwendung eines Mittels, das zur Lyse einer perikardialen/epikardialen Adhäsion fähig ist, zur Herstellung eines Medikaments zur Verwendung in einem Verfahren des Zugänglichmachens des perikardialen Raums, bevor ein therapeutisches Mittel in den perikardialen Raum verabreicht wird, wobei das Verfahren des Zugänglichmachens des perikardialen Raums weiter den Schritt des Expandierens des perikardialen Raums umfasst.

50. Verwendung nach Anspruch 49, wobei das Expandieren des perikardialen Raums die temporäre Verabreichung einer Flüssigkeit oder eines Gases umfasst.

51. verwendung nach Anspruch 49 oder 50, wobei das Mittel, das zur Lyse einer perikardialen/epikardialen Adhäsion fähig ist, ein Mittel, ausgewählt aus einer Gruppe, bestehend aus irgendeinem fibrinolytischen Mittel, Gewebe-Plasminogen-Aktivator (tPA), Streptokinase, Urokinase, Kollagenase und einer Matrix-Metallprotease, ist.

52. Verwendung nach Anspruch 49, wobei das therapeutische Mittel in einem der Ansprüche 3 bis 24 definiert wird.

53. Verwendung nach Anspruch 1, wobei das erste therapeutische Mittel eine L-Aminosäure-Decarboxylase umfasst.

54. Verwendung nach Anspruch 53, wobei die pharmazeutische Zusammensetzung weiter ein zweites therapeutisches Mittel, ausgewählt aus einer Gruppe, bestehend aus L-Dopa, Tyrosin, einem Polynukleotid das für Tyrosin-Hydroxylase codiert und einem Tyrosin-Hydroxylase-Peptid, umfasst.

55. Verwendung nach Anspruch 1, wobei das erste therapeutische Mittel den vaskulären epithelialen Wachstumsfaktor (VEGF), ein biologisch aktives Fragment davon oder ein Chimäres davon, umfasst.

## Revendications

1. Utilisation d'un premier agent thérapeutique dans la fabrication d'une composition pharmaceutique pour le traitement ou la prévention d'une affection coronarienne, dans laquelle la composition pharmaceutique est administrée dans l'espace péricardique d'un patient simultanément à, ou de façon séquentielle après, l'augmentation de l'accès au tissu myocardique du premier agent thérapeutique.

2. Utilisation d'un premier agent thérapeutique dans la fabrication d'une composition pharmaceutique pour le traitement ou la prévention d'une affection coronarienne, dans laquelle la composition pharmaceutique est administrée dans l'espace péricardique d'un patient et la composition pharmaceutique comprend un agent qui augmente l'accès au tissu myocardique.

3. Utilisation selon la revendication 1 ou 2, dans laquelle la composition pharmaceutique comprend en outre un second agent thérapeutique.

4. Utilisation selon la revendication 1, 2 ou 3, dans laquelle le premier et/ou le second agent thérapeutique est choisi parmi un polypeptide, un polynucléotide, une petite molécule organique, un peptide et un peptoïde.

5. Utilisation selon la revendication 1, 2 ou 3, dans laquelle le premier et/ou le second agent thérapeutique comprend un polypeptide facteur de croissance des fibroblastes (FGF) ou un polypeptide facteur de croissance analogue à l'insuline I (IGF-1).

6. Utilisation selon la revendication 5, dans laquelle le polypeptide FGF est choisi parmi bFGF, aFGF, et FGF-5.

7. Utilisation selon la revendication 1, 2 ou 3, dans laquelle le premier et/ou le second agent thérapeutique est choisi parmi un agent anti-apoptotique, un agent thrombolytique, un agent pro-angiogénique, un agent anti-arhytmique, un agent améliorant la contractilité, un inhibiteur de complément, un inhibiteur de lésion par reperfusion, un inhibiteur calcique, un bêta-bloquant, un réducteur de postcharge, un réducteur de précharge, un agent vaso-actif, un agent antithrombotique, un agent antiplaquettaire, un agent antiprolifératif, un agent anti-inflammatoire, un agent immunomodulateur, un agent immunosuppresseur, un inhibiteur de métabolites oxygénés réactifs, un agent antiangiogénique, un facteur de croissance des myocytes, un agent vasoactif, un agent cardioprotecteur, un agent chélatant le fer, un agent antihypertenseur, un agent anti-intégrine, un agent pro-apoptotique, un agent antiviral, un agent antiparasitaire, un piégeur de radicaux libres, un agent antitumoral, une protéine qui peut être déficiente ou négativement régulée pendant le développement d'une cardiomyopathie, et leurs dérivés biologiquement actifs.

8. Utilisation selon la revendication 7; dans laquelle la protéine qui peut être déficiente ou négativement régulée pendant le développement d'une cardiomyopathie est la troponine C.

9. Utilisation selon la revendication 7, dans laquelle le premier et/ou le second agent thérapeutique est un agent antitumoral et l'agent antitumoral est choisi parmi un agent chimiothérapeutique, un agent de sensibilisation à l'irradiation et un implant radioactif.

10. Utilisation selon la revendication 1, 2 ou 3, dans laquelle le premier et/ou le second agent thérapeutique est un inhibiteur.

11. Utilisation selon la revendication 10, dans laquelle l'inhibiteur est choisi parmi un inhibiteur de synthèse ou de dépôt de lipides ou de cholestérol, un inhibiteur de recrutement ou d'activation de macrophages ou de cellules inflammatoires, un inhibiteur de microtubules, un agent anti-inflammatoire, un agent antithrombotique, un agent antiplaquettaire et un inhibiteur de prolifération néointimale.

12. Utilisation selon la revendication 10, dans laquelle l'inhibiteur est un inhibiteur de NFκB.

13. Utilisation selon la revendication 12, dans laquelle l'inhibiteur de NFκB est choisi parmi IKB, PDTC, des chélateurs de métaux NAC et des antioxydants.

14. Utilisation selon la revendication 10, dans laquelle l'inhibiteur est choisi parmi une protéine, un peptide, un oligopeptide ou une petite molécule.

15. Utilisation selon la revendication 7, dans laquelle le premier et/ou le second agent thérapeutique est un agent anti-prolifératif et l'agent anti-prolifératif est choisi parmi un ribozyme, un oligonucléotide antisens, un anticorps, un inhibiteur contre l'un parmi c-myb, ras, raf, la kinase de P13 et la cycline, une protéine suicide, un gène suicide, une protéine pro-apoptotique et un gène pro-apoptotique.

16. Utilisation selon la revendication 7, dans laquelle le premier et/ou le second agent thérapeutique est une protéine pro-apoptotique et la protéine pro-apoptotique est choisie parmi fas, fab et l'enzyme de conversion de l'interleukin 1b.

17. Utilisation selon la revendication 7, dans laquelle le premier et/ou le second agent thérapeutique est un agent anti-angiogénique et l'agent anti-angiogénique est choisi parmi le facteur 4, la thrombospondine, un inhibiteur tissulaire d'une métalloprotéase, l'angiostatine, TGF-β, l'interféron-α, une protéine apparentée à la proliférine, l'un de leurs fragments biologiquement actifs et l'une de leurs chimères.

18. Utilisation selon la revendication 7, dans laquelle le premier et/ou le second agent thérapeutique est un antibiotique et l'antibiotique est choisi parmi un agent antiviral, un agent antitrypanosomal, un agent antibactérien et un agent antifongique.

19. Utilisation selon la revendication 7, dans laquelle le premier et/ou le second agent thérapeutique est un agent immunomodulateur et l'agent immunomodulateur est choisi parmi un agent cytotoxique, un stéroïde, la cyclosporine et un inhibiteur de complément.

20. Utilisation selon la revendication 19, dans laquelle l'agent immunomodulateur est un inhibiteur de complément et l'inhibiteur de complément est choisi parmi DAF, CAB2, l'un de leurs fragments biologiquement actifs et l'une de leurs chimères.

21. Utilisation selon la revendication 7, dans laquelle le premier et/ou le second agent thérapeutique est un agent anti-inflammatoire et l'agent anti-inflammatoire est choisi parmi un stéroïde, un agent anti-inflammatoire non stéroïdien, la cyclosporine, un agent chimiothérapeutique et un inhibiteur de complément.

22. Utilisation selon la revendication 7, dans laquelle le premier et/ou le second agent thérapeutique est un agent anti-arythmique et l'agent anti-arythmique est choisi parmi l'adénosine, la quinidine, le propranolol, la digoxine, la lidocaïne, le brétylium, l'amiodarone, et le verapamil.

23. Utilisation selon la revendication 7, dans laquelle le premier et/ou le second agent thérapeutique est un agent antihypertenseur, et l'agent antihypertenseur est choisi parmi l'hydralazine, le propanol, le peptide natriurétique auriculaire et un antagoniste d'endothéline.

24. Utilisation selon la revendication 1, 2 ou 3, dans laquelle le premier et/ou le second-agent thérapeutique est un polypeptide choisi parmi l'activateur tissulaire du plasminogène (tPA), un inhibiteur de l'enzyme de conversion de l'interleukine 1β, l'activateur de type urokinase du plasminogène (uPA), l'urokinase, la streptokinase, un inhibiteur d'inhibiteur de plasmine α-2, un inhibiteur d'inhibiteur de l'activateur du plasminogène 1 (PAI-1), le facteur de croissance des fibroblastes basique (VEGF), l'angiogénine, le facteur de croissance transformant α (TGF-α), le facteur de croissance transformant β (TGF-β), le facteur nécrosant des tumeurs α (TNF-α), le facteur de croissance dérivé des plaquettes (PDGF), le facteur de croissance placentaire (PGF), le facteur de croissance des hépatocytes, la proliférine, la protéine membranaire DAF, CAB-2, l'inhibiteur de la voie du facteur tissulaire (TFPI), l'héparine, l'hirudine, la protéine C, la protéine S, l'antithrombine III, le peptide anticoagulant de tique (TAP), l'antistasine, l'antagoniste de la glycoprotéine IIb/IIa, des anticorps, la thymidine kinase d'Herpes, fas, faf, le facteur plaquettaire 4, la thrombospondine, un inhibiteur tissulaire d'une métalloprotéase, la prolactine, le récepteur soluble de bFGF, une protéine apparentée à la proliférine, le facteur de croissance des myocytes, la superoxyde dismutase (SOD), la troponine C, le récepteur bêta-adrénergique, le facteur de croissance analogue à l'insuline I (IGF-1), la protéine anticoagulante de nématode (NAP), leurs fragments biologiquement actifs, et leurs chimères.

25. Utilisation selon l'une quelconque des revendications 1 à 24, dans laquelle l'administration dans l'espace péricardique est effectuée par entrée interne ou entrée externe.

26. Utilisation selon la revendication 25, dans laquelle la méthode d'entrée interne est choisie parmi une entrée par l'oreillette gauche, une entrée par le ventricule droit et une entrée par le ventricule gauche.

27. Utilisation selon la revendication 25, dans laquelle la méthode d'entrée externe est choisie parmi un protocole à thorax ouvert, une chirurgie invasive minimale (MIS) et une entrée percutanée.

28. Utilisation selon la revendication 27, dans laquelle l'entrée percutanée est facilitée par un dispositif choisi parmi une aiguille, un cathéter, une canule et un trocar.

29. Utilisation selon l'une quelconque des revendications 1 à 24, dans laquelle l'administration dans l'espace péricardique est effectuée par un protocole choisi parmi une injection, une cathétérisation, la création de canaux de perfusion crées par laser, une canulation, l'utilisation d'un canon à particules et l'utilisation d'une pompe.

30. Utilisation selon l'une quelconque des revendications précédentes, dans laquelle l'affection coronarienne est une affection choisie parmi une occlusion de l'artère coronaire, des syndromes ischémiques, une cardiomyopathie, une arythmie, une dysrhytmie, une infection, et un état inflammatoire.

31. Utilisation selon la revendication 30, dans laquelle l'affection coronarienne est une occlusion de l'artère coronaire et l'occlusion de l'artère coronaire est due ou est associée à un dépôt de lipide/cholestérol, un recrutement de macrophages/cellules inflammatoires, une rupture de plaque, une thrombose, un dépôt de plaquettes ou une prolifération néointimale.

32. Utilisation selon la revendication 30, dans laquelle l'affection coronarienne est un syndrome ischémique et le syndrome ischémique est dû ou est associé à un infarctus du myocarde, un angor stable, un angor instable, une resténose de l'artère coronaire ou une lésion par reperfusion.

33. Utilisation selon la revendication 30, dans laquelle l'affection coronarienne est une cardiomyopathie et la cardiomyopathie est due ou est associée à un syndrome ischémique, une cardiotoxine, une infection, une hypertension, une maladie métabolique, une irradiation, une maladie neuromusculaire, un trauma ou une cause idiopathique.

34. Utilisation selon la revendication 33, dans laquelle la cardiomyopathie est due à une maladie infiltrante, et la maladie infiltrante est choisie parmi une sarcoïdose, une hémochromatose, une amyloïdose, la maladie de Fabry et le syndrome de Hurler.

35. Utilisation selon la revendication 33, dans laquelle la cardiomyopathie est due à une maladie métabolique et la maladie métabolique est choisie parmi une urémie, le béribéri, et une maladie de stockage du glycogène.

36. Utilisation selon la revendication 30, dans laquelle l'affection coronarienne est une arythmie ou une disrythmie due ou associée à un syndrome ischémique, une cardiotoxine adromycine, une infection, une hypertension, une maladie métabolique, une irradiation, une maladie neuromusculaire, une maladie infiltrante, un trauma ou une cause idiopathique.

37. Utilisation selon la revendication 30, dans laquelle l'affection coronarienne est une infection provoquée par un agent pathogène choisi parmi une bactérie, un virus, un champignon, et un parasite.

38. Utilisation selon la revendication 30, dans laquelle l'affection coronarienne est une affection inflammatoire et l'affection inflammatoire est associée à une myocardite, une péricardite, une endocardite, un rejet cardiaque immunitaire et des affections inflammatoires dues à l'une parmi une maladie idiopathique, auto-immune ou du tissu conjonctif.

39. Utilisation selon l'une quelconque des revendications précédentes, dans laquelle l'augmentation de l'accès comprend une étape choisie parmi l'augmentation de la pénétration du tissu myocardique par le premier et/ou le second agent thérapeutique et la création de canaux de perfusion.

40. Utilisation selon la revendication 39, dans laquelle l'augmentation de la pénétration comprend une administration d'un facteur pro-angiogénique dans l'espace péricardique d'un patient pour augmenter la vascularisation du tissu myocardique.

41. Utilisation selon la revendication 39, dans laquelle l'augmentation de la pénétration comprend l'administration du premier et/ou du second agent thérapeutique dans une formulation qui augmente la pénétration dans le tissu du premier et/ou du second agent thérapeutique.

42. Utilisation selon l'une quelconque des revendications précédentes, dans laquelle la composition pharmaceutique comprend un composant choisi parmi un liposome, un liposome de cyclodextrine, un liposome hétérovésiculaire, une vésicule membranaire synthétique, un gel, un polymère, un excipient, des matrices, une particule chargée et un tampon.

43. Utilisation selon la revendication 42, dans laquelle le composant est un gel et le gel comprend du Focalgel.

44. Utilisation selon la revendication 42 dans laquelle le composant est un liposome et le liposome comprend du Depofoam®.

45. Utilisation d'un agent thérapeutique dans la fabrication d'une composition pharmaceutique destinée à être utilisée dans une méthode de traitement du tissu de muscle cardiaque, cette méthode comprenant le fait :
a. d'identifier une zone d'infarctus ou ischémique,
b. d'accéder à un espace péricardique dans la région de la zone d'infarctus ou ischémique ; et
c. de délivrer la composition pharmaceutique dans la région de la zone d'infarctus ou ischémique,
dans laquelle l'accès de l'agent thérapeutique à la région de la zone d'infarctus ou ischémique est augmenté par la création de canaux de perfusion, l'administration d'un facteur pro-angiogénique dans l'espace péricardique ou l'inclusion dans la composition pharmaceutique d'un agent qui augmente la pénétration dans le tissu de l'agent thérapeutique.

46. Utilisation selon la revendication 45, dans laquelle la composition pharmaceutique comprend en outre un composant choisi parmi un gel adhérent, un polymère, un ligand de ciblage, un anticorps de ciblage et un agent actif à pH faible.

47. Utilisation selon la revendication 45 ou 46, dans laquelle la composition pharmaceutique comprend une combinaison d'au moins deux agents thérapeutiques.

48. Utilisation selon la revendication 45, 46 ou 47, dans laquelle l'agent thérapeutique est tel que défini dans l'une quelconque des revendications 3 à 24.

49. Utilisation d'un agent capable de lyser une adhésion péricardique/épicardique pour la fabrication d'un médicament destiné à être utilisé dans une méthode d'accession à l'espace péricardique avant l'administration d'un agent thérapeutique dans l'espace péricardique, dans laquelle la méthode d'accession à l'espace péricardique comprend en outre l'étape d'expansion de l'espace péricardique.

50. Utilisation selon la revendication 49, dans laquelle l'expansion de l'espace péricardique comprend l'administration temporaire d'un liquide ou d'un gaz.

51. Utilisation selon la revendication 49 ou 50, dans laquelle l'agent capable de lyser une adhésion péricardique/épicardique est un agent choisi parmi tout agent fibrinolytique, l'activateur tissulaire du plasminogène (TPA), la streptokinase, l'urokinase, la collagénase, et une métalloprotéase de matrice.

52. Utilisation selon la revendication 49, dans laquelle l'agent thérapeutique est tel que défini dans l'une quelconque des revendications 3 à 24.

53. Utilisation selon la revendication 1, dans laquelle le premier agent thérapeutique comprend une acide aminé L décarboxylase.

54. Utilisation selon la revendication 53, dans laquelle la composition pharmaceutique comprend en outre un second agent thérapeutique choisi parmi la L-Dopa, la tyrosine, un polynucléotide codant une tyrosine hydroxylase, et un polypeptide tyrosine hydroxylase.

55. Utilisation selon la revendication 1, dans laquelle le premier agent thérapeutique comprend un facteur de croissance épithélial vasculaire (VEGF), l'un de ses fragments biologiquement actifs ou l'une de ses chimères.
